# EUROPEAN PATENT SPECIFICATION

(11) **EP 3 039 429 B1**
(45) Date of publication and mention of the grant of the patent: **10.10.2018**
(21) Application number: 14776728.9
(22) Date of filing: 28.08.2014
(51) Int. Cl.: G01N 33/574

(54) **POLYP RECURRENCE**
WIEDERAUFTRETEN VON POLYPEN
RÉAPPARITION DE POLYPES

(30) Priority: 30.08.2013 US 201361872613 P
(43) Date of publication of application: 06.07.2016
(73) Proprietor: Nestec S.A., 1800 Vevey (CH)
(72) Inventor: SINGH, Sharat, Rancho Santa Fe California 92127 (US); HOE, Nicholas, San Diego California 92129 (US); HESTER, Kelly D., San Diego California 92121 (US)
(74) Representative: Krishnan, Sri
(86) International application number: PCT/IB2014/064135
(87) International publication number: WO 2015/028974

(56) References cited:
- EP-A1- 2 589 666
- J LEE ET AL: "A Novel Proteomics-Based Clinical Diagnostics Technology Identifies Heterogeneity in Activated Signaling Pathways in Gastric Cancers", PLOS ONE, vol. 8, no. 1, 25 January 2013 (2013-01-25), page e54644, XP055134461,
- A MUNAKATA ET AL: "Colonoscopic Polypectomy with Local Injection of Methylene Blue", TOHOKU J. EXP. MED., vol. 173, 1994, pages 377-382, XP055157722,
- H NEUMANN ET AL: "Endocytoscopy-Based Detection of Focal High-Grade Intraepithelial Neoplasia in Colonic Polyps", CLINICAL GASTROENTEROLOGY AND HEPATOLOGY, vol. 9, no. 2, 2011, page e13, XP028187335,
- T TAKAYAMA ET AL: "Aberrant Crypt Foci: Detection, Gene Abnormalities, and Clinical Usefulness", CLINICAL GASTROENTEROLOGY AND HEPATOLOGY, vol. 3, no. 7, 2005, pages S42-S45, XP005120826,
- PATEL B B ET AL: "Age-related increase in colorectal cancer stem cells in macroscopically normal mucosa of patients with adenomas: A risk factor for colon cancer", BIOCHEMICAL AND BIOPHYSICAL RESEARCH COMMUNICATIONS, vol. 378, no. 3, 2009, pages 344-347, XP025837821,
- G BONGERS ET AL: "A Role for the Epidermal Growth Factor Receptor Signaling in Development of Intestinal Serrated Polyps in Mice and Humans", GASTROENTEROLOGY, vol. 143, no. 3, 2012, pages 730-740, XP055156117,
- L KAKLAMANIS ET AL: "Interleukin-4 receptor and epidermal growth factor receptor expression in colorectal cancer", BRITISH JOURNAL OF CANCER, vol. 66, no. 4, 1992, pages 712-716, XP055156316,
- INGRID LJUSLINDER ET AL: "Increased epidermal growth factor receptor expression at the invasive margin is a negative prognostic factor in colorectal cancer", INTERNATIONAL JOURNAL OF CANCER, vol. 128, no. 9, 2011, pages 2031-2037, XP055157415,
- G BAIOCCHI ET AL: "ErbB family immunohistochemical expression in colorectal cancer patients with higher risk of recurrence after radical surgery", INTERNATIONAL JOURNAL OF COLORECTAL DISEASE, vol. 24, no. 9, 2009, pages 1059-1068, XP019739599,
- DATABASE MEDLINE [Online] US NATIONAL LIBRARY OF MEDICINE (NLM), BETHESDA, MD, US; 1997, MALECKA-PANAS E ET AL: "Differential activation of total and EGF receptor (EGF-R) tyrosine kinase (tyr-k) in the rectal mucosa in patients with adenomatous polyps, ulcerative colitis and colon cancer.", XP002733573, Database accession no. NLM9164515

## Description

### CROSS-REFERENCE TO RELATED APPLICATION

The present application claims priority to U.S. Provisional Patent Application No. 61/872,613, filed August 30, 2013.

### BACKGROUND OF THE INVENTION

Colorectal cancer (CRC) is the third most common cancer worldwide after lung and breast cancers, with almost 60% of all colorectal cancers occurring in the more developed regions. It is estimated that there were over 140,000 new cases diagnosed in the United States in 2013 (American Cancer Society: Cancer Facts and Figures 2013. Atlanta, GA: American Cancer Society, 2013). The majority of people who develop colorectal cancer have no known risk factors. Although the exact cause of colorectal cancer is not known, factors such as age, personal history, and the presence of adenomas increase a person's risk of developing the disease.

The risk of developing CRC also increases with age. The disease is most common in people over the age of 50, and the chance of acquiring CRC increases with each decade. However, colorectal cancer can develop in younger people as well.

Research shows that women who have a history of ovarian, uterine, or breast cancer have a somewhat higher risk of developing colorectal cancer. A person who already has had colorectal cancer may develop the disease a second time, especially if the first disease was diagnosed before the age of 60. In addition, people who have chronic inflammatory conditions of the colon, such as inflammatory bowel disease including ulcerative colitis or Crohn's disease, are at higher risk of developing CRC.

Colorectal cancer is believed to develop predominantly in cases involving non-malignant precursor lesions or adenomatous polyps (also called adenomas). At a certain point, the adenoma can invade the sub-mucosa and become malignant. It can progress from localized (Stage I) to metastasized (Stage IV) cancer. Invasive cancers that are confined within the wall of the colon (Stage I and II) are often curable with surgery. However, if left untreated, the cancer can spread to regional lymph nodes (Stage III), with approximately 48% of patients diagnosed at this stage surviving longer than five years. Cancer that has metastasized to other parts of the body (Stage IV) is usually not curable, with approximately 7% surviving longer than five years.

Colorectal polyps can be non-cancerous growths on the inner wall of the colon and/or rectum. While they are fairly common in people over 50, adenomas increase the risk of developing colorectal cancer. Even though adenomas are non-cancerous polyps, they are considered precursors to developing colon and rectal cancer.

The likelihood that an adenoma will develop or has already developed into cancer is partially dependent on its size. Typically, a larger polyp is more likely to be or become malignant. In addition, the malignant potential of an adenoma is related to the organizational structure of the cells in the polyp. The cells within the polyp also vary in their tendency to become cancerous (malignant). It has been shown that the presence of multiple polyps increases the likelihood of developing CRC. For instance, patients with multiple non-cancerous polyps are more likely to develop additional polyps in the future that may eventually become malignant.

Most adenomatous polyps are considered sporadic and thus not associated to an inherited gene mutation. Nevertheless, the risk of having colon polyps >1 cm in size or developing colon cancer is two-fold greater if a first degree relative has colon polyps >1 cm in size. In one study, the risk increased from about 4% to 8%. Therefore, there is likely to be a genetic factor even in sporadic adenomas.

Kaklamanis et al (1992) Br. J. Cancer 66, 712-716 performed EGFR staining of colonic polyps and found EGFR expression correlated with turmour progression. Patel et al (2009) Biochem. Biophys.Res. Comm. 378, 344-347 demonstrated a linear increase in number of adenomas with aging acompanied by a parallel rise in EGFR expression in the normal appearing colonic mucosa.

Current methods for diagnosing colorectal cancer are unable to determine whether a benign or pre-cancerous polyp is likely to progress towards cancer and become malignant. There is a need for accurate colon cancer staging of polyp tissues. The present invention fulfills this and other needs by providing a method for determining whether a patient is at risk of developing colorectal cancer from a polyp sample.

### BRIEF SUMMARY OF THE INVENTION

In one embodiment, the present invention provides a method for diagnosing (*e.g*., determining) the risk of developing colorectal cancer (CRC) in a subject or identifying a subject as likely to develop colorectal cancer (CRC), the method comprising:
a) lysing a cell from a polyp sample taken from the subject to form a cell lysate;
b) measuring the activation and/or expression level of at least one signal transduction analyte in the cell lysate, wherein the at least one signal transduction analyte is HER1; and
c) indicating whether the subject is at risk of developing colorectal cancer (CRC) based upon the activation and/or expression level of the at least one signal transduction analyte compared to that of a control

In some embodiments, the at least one signal transduction analyte further includes one or more signal transduction analytes selected from the group consisting of HER2, HER3, cMET, PI3K, IGF1R, SHC, CK, AKT, ERK, MEK, RSK, PRAS, RPS6, and a combination thereof.

In some embodiments, the subject is at risk of developing CRC when the measured activation and/or expression level of the at least one signal transduction analyte is higher compared to that of a control.

In some embodiments, step (b) comprises measuring the activation and/or expression level of any combination of two, three, four, five, six, seven, eight, nine, ten, eleven, twelve, thirteen, or fourteen of the signal transduction analytes.

In some embodiments, step (b) is performed with a proximity dual detection assay. In some aspects, the proximity dual detection assay is a Collaborative Enzyme Enhanced Reactive ImmunoAssay (CEER).

In some embodiments, the activation level of the at least one signal transduction analyte corresponds to the phosphorylation level thereof. In some embodiments, the activation level of the at least one signal transduction analyte corresponds to the phosphorylation level of HER1, HER2, HER3, cMET, PI3K, IGF1R, SHC, CK, AKT, ERK, MEK, RSK, PRAS, RPS6 and a combination thereof In some embodiments, the activation level of the at least one signal transduction analyte corresponds to a level of a PI3K complex.

In some embodiments, the control is from a healthy subject. In some embodiments, the control is from a CRC subject. In some embodiments, the control is from a non-adenomatous tissue.

In some embodiments, the polyp sample is from a polypectomy. In some embodiments, the polyp sample is an adenomatous polyp. In some embodiments, the polypectomy is a polypectomy of the colon and/or rectum.

Other objects, features, and advantages of the present invention will be apparent to one of skill in the art from the following detailed description and figures.

### BRIEF DESCRIPTION OF THE DRAWINGS

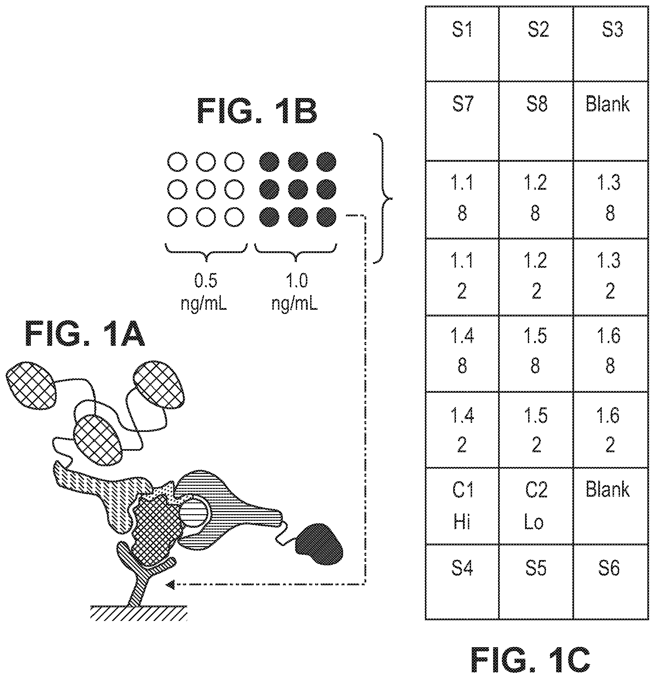

FIGS. 1A-1C show exemplary embodiments of the CEER assay used to measure the level of activated and total analytes such as HER1, HER2, HER3, cMET, PI3K, IGF1R, SHC, CK, Akt, Erk, Mek, Rsk, PRAS and RPS6. FIG. 1A shows one embodiment of the proximity dual detecting assay format of the invention (*e.g.,* CEER), which relies on the co-localization of two additional detector antibodies linked with enzymes for subsequent channeling events per each target protein bound. FIG. 1B shows the spatial arrangement of the capture antibodies printed on a substrate such as a nitrocellulose-coated glass slide. The capture antibodies are printed in triplicate and at serial dilution concentrations of 1 mg/ml and 0.5 mg/ml. FIG. 1C shows that multiple analytes and samples can be assayed on a slide.

FIGS. 2A-2H show the levels of activated analytes in normal patient samples, polyp samples in Group A, and polyp samples in Group B. pHER1 levels are presented in FIG. 2A; pHER2 in FIG. 2B; pHER3 in FIG. 2C; pPBK in FIG. 2D; pAKT in FIG. 2E;pERK in FIG. 2F; pMEK in FIG. 2G; and pRSK in FIG. 2H.

FIGS. 3A-3B show the level of activated analytes in polyp samples in Group A (FIG. 3A) and Group B (FIG. 3B). Samples in Group B have a higher level of pHER3 compared to samples in Group A.

### DETAILED DESCRIPTION OF THE INVENTION

### I. Introduction

The present invention provides assay and methods for predicting a patient's risk of developing CRC by measuring the activation and/or expression level of analytes in a polyp sample. The methods are *in vitro* methods. The present disclosure also provides assays and methods for selecting a suitable anticancer drug for the treatment of colorectal cancer. In particular embodiments, the methods rely on the detection of the activation state or the level of a specific combination of signal transduction analytes in a cell from a polyp sample. Thus, the methods described herein are particularly useful for diagnosing CRC in a subject at an early stage of cancer, for example, in a subject with pre-malignant polyps or adenomas.

Example 1 demonstrates that pre-cancerous polyps have higher levels of activated (phosphorylated) analytes such as HER1, HER2, HER3, PBK, AKT, ERK, MEK, RSK and a combination thereof, compared to normal controls and non-cancerous polyps.

### II. Definitions

The term "cancer" is intended to include any member of a class of diseases characterized by the uncontrolled growth of aberrant cells. The term includes all known cancers and neoplastic conditions, whether characterized as malignant, benign, soft tissue, or solid, and cancers of all stages and grades including pre- and post-metastatic cancers. Examples of different types of cancer include, but are not limited to, digestive and gastrointestinal cancers such as colorectal cancer, gastric cancer (*e.g.,* stomach cancer), gastrointestinal stromal tumors (GIST), gastrointestinal carcinoid tumors, colon cancer, rectal cancer, anal cancer, bile duct cancer, small intestine cancer, and esophageal cancer; breast cancer; lung cancer (*e.g.,* non-small cell lung cancer (NSCLC)); gallbladder cancer; liver cancer; pancreatic cancer; appendix cancer; prostate cancer, ovarian cancer; renal cancer (*e.g.,* renal cell carcinoma); cancer of the central nervous system; skin cancer; lymphomas; gliomas; choriocarcinomas; head and neck cancers; osteogenic sarcomas; and blood cancers. As used herein, a "tumor" comprises one or more cancerous cells.

The term "colon cancer staging" includes the TNM (tumors/nodes/metastases) system from the American Joint Committee on Cancer as well as the broader Stage I-IV groupings. Stage 0 or T is corresponds to a colon tumor confined to the mucosa. Stage I or T1 corresponds to a colon tumor that invades the submucosa. Stage I or T2 corresponds to a colon tumor that invades the muscularis propria. Stage II-A or T3 corresponds to a colon tumor that invades the subserosa or beyond, though without involved of other organs. Stage II-B or T4 indicates that a colon tumor invades adjacent organs or perforates the viscreal peritoneum. Stage III-A or T1-2 N1 corresponds to colon tumor metastasis to 1-3 regional lymph nodes. Stage III-B or T3-4 N2 corresponds to colon tumor metastasis to 4 or more regional lymph nodes. Stage IV or any T, any N, and M1 corresponds to the presence of distance metastasis.

The term "analyte" includes any molecule of interest, typically a macromolecule such as a polypeptide, whose presence, amount (expression level), activation state, and/or identity is determined.

The term "signal transduction molecule" or "signal transducer" includes proteins and other molecules that carry out the process by which a cell converts an extracellular signal or stimulus into a response, typically involving ordered sequences of biochemical reactions inside the cell. Examples of signal transduction molecules include, but are not limited to, receptor tyrosine kinases such as EGFR (*e.g.,* EGFR/HER1/ErbB1, HER2/Neu/ErbB2, HER3/ErbB3, HER4/ErbB4), VEGFR1/FLT1, VEGFR2/FLK1/KDR, VEGFR3/FLT4, FLT3/FLK2, PDGFR (*e.g.,* PDGFRA, PDGFRB), c-KIT/SCFR, INSR (insulin receptor), IGF-IR, IGF-IIR, JRR (insulin receptor-related receptor), CSF-1R, FGFR 1-4, HGFR 1-2, CCK4, TRK A-C, c-MET, RON, EPHA 1-8, EPHB 1-6, AXL, MER, TYRO3, TIE 1-2, TEK, RYK, DDR 1-2, RET, c-ROS, V-cadherin, LTK (leukocyte tyrosine kinase), ALK (anaplastic lymphoma kinase), ROR 1-2, MUSK, AATYK 1-3, and RTK 106; truncated forms of receptor tyrosine kinases such as truncated HER2 receptors with missing amino-terminal extracellular domains (*e.g.,* p95ErbB2 (p95m), p110, p95c, p95n, *etc.*), truncated cMET receptors with missing amino-terminal extracellular domains, and truncated HER3 receptors with missing amino-terminal extracellular domains; receptor tyrosine kinase dimers (*e.g.,* p95HER2/HER3; p95HER2/HER2; truncated HER3 receptor with HER1, HER2, HER3, or HERA; HER2/HER.2; HER3/HER3; HER2/HER3; HER1/HER2; HER1/HER3; HER2/HER4; HER3/HER4; *etc.*); non-receptor tyrosine kinases such as BCR-ABL, Src, Frk, Btk, Csk, Abl, Zap70, Fes/Fps, Fak, Jak, Ack, and LIMK; tyrosine kinase signaling cascade components such as AKT (*e.g.,* AKT1, AKT2, AKT3), MEK (MAP2K1), ERK2 (MAPK1), ERK1 (MAPK3), PI3K (*e.g.,* PIK3CA (p110), PIK3R1 (p85)), PDK1, PDK2, phosphatase and tensin homolog (PTEN), SGK3, 4E-BP1, P70S6K (*e*.*g*., p70 S6 kinase splice variant alpha I), protein tyrosine phosphatases (*e.g.,* PTP1B, PTPN13, BDP1, *etc*.), RAF, PLA2, MEKK, JNKK, JNK, p38, Shc (p66), Ras (*e.g.,* K-Ras, N-Ras, H-Ras), Rho, Rac1, Cdc42, PLC, PKC, p53, cyclin D1, STAT1, STAT3, phosphatidylinositol 4,5-bisphosphate (PIP2), phosphatidylinositol 3,4,5-trisphosphate (PIP3), mTOR, BAD, p21, p27, ROCK, IP3, TSP-1, NOS, GSK-3p, RSK 1-3, JNK, c-Jun, Rb, CREB, Ki67, and paxillin; nuclear hormone receptors such as estrogen receptor (ER), progesterone receptor (PR), androgen receptor, glucocorticoid receptor, mineralocorticoid receptor, vitamin A receptor, vitamin D receptor, retinoid receptor, thyroid hormone receptor, and orphan receptors; nuclear receptor coactivators and repressors such as amplified in breast cancer-1 (AIB1) and nuclear receptor corepressor 1 (NCOR), respectively; and combinations thereof.

The term "activation state" refers to whether a particular signal transduction molecule is activated. Similarly, the term "activation level" refers to what extent a particular signal transduction molecule is activated. The activation state typically corresponds to the phosphorylation, ubiquitination, and/or complexation status of one or more signal transduction molecules. Non-limiting examples of activation states (listed in parentheses) include: HER1/EGFR (EGFRvIII, phosphorylated (p-) EGFR, EGFR:Shc, ubiquitinated (u-) EGFR, p-EGFRvIII); ErbB2 (p-ErbB2, p95HER2 (truncated ErbB2), p-p95HER2, ErbB2:Shc, ErbB2:PI3K, ErbB2:EGFR, ErbB2:ErbB3, ErbB2:ErbB4); ErbB3 (p-ErbB3, truncated ErbB3, ErbB3:PI3K, p-ErbB3:PI3K, ErbB3:Shc); ErbB4 (p-ErbB4, ErbB4:Shc); c-MET (p-c-MET, truncated c-MET, c-Met:HGF complex); AKT1 (p-AKT1); AKT2 (p-AKT2); AKT3 (p-AKT3); PTEN (p-PTEN); P70S6K (p-P70S6K); MEK (p-MEK); ERK1 (p-ERK1); ERK2 (p-ERK2); PDK1 (p-PDK1); PDK2 (p-PDK2); SGK3 (p-SGK3); 4E-BP1 (p-4E-BP1); PIK3R1 (p-PIK3R1); c-KIT (p-c-KIT); ER (p-ER); IGF-1R (p-IGF-1R, IGF-1R:IRS, IRS:PI3K, p-IRS, IGF-1R:PI3K); INSR (p-INSR); FLT3 (p-FLT3); HGFR1 (p-HGFR1); HGFR2 (p-HGFR2); RET (p-RET); PDGFRA (p-PDGFRA); PDGFRB (p-PDGFRB); VEGFR1 (p-VEGFR1, VEGFR1:PLCγ, VEGFR1:Src); VEGFR2 (p-VEGFR2, VEGFR2:PLCγ, VEGFR2:Src, VEGFR2:heparin sulphate, VEGFR2:VE-cadherin); VEGFR3 (p-VEGFR3); FGFR1 (p-FGFR1); FGFR2 (p-FGFR2); FGFR3 (p-FGFR3); FGFR4 (p-FGFR4); TIE1 (p-TIE1); TIE2 (p-TIE2); EPHA (p-EPFIA); EPHB (p-EPHB); GSK-3β (p-GSK-3β); NFKB (p-NFKB), IKB (p-IKB, p-P65:IKB); BAD (p-BAD, BAD: 14-3-3); mTOR (p-mTOR); Rsk-1 (p-Rsk-1); Jnk (p-Jnk); P38 (p-P38); STAT1 (p-STAT1); STAT3 (p-STAT3); FAK (p-FAK); RB (p-RB); Ki67; p53 (p-p53); CREB (p-CREB); c-Jun (p-c-Jun); c-Src (p-c-Src); paxillin (p-paxillin); GRB2 (p-GRB2), She (p-Shc), Ras (p-Ras), GAB1 (p-GAB1), SHP2 (p-SHP2), GRB2 (p-GRB2), CRKL (p-CRKL), PLCγ (p-PLCγ), PKC (*e.g.,* p-PKCα, p-PKCβ, p-PKCδ), adducin (p-adducin), RB1 (p-RB1), and PYK2 (p-PYK2).

As used herein, the term "dilution series" is intended to include a series of descending concentrations of a particular sample (*e.g.,* cell lysate) or reagent (*e*.*g*., antibody). A dilution series is typically produced by a process of mixing a measured amount of a starting concentration of a sample or reagent with a diluent (*e.g.,* dilution buffer) to create a lower concentration of the sample or reagent, and repeating the process enough times to obtain the desired number of serial dilutions. The sample or reagent can be serially diluted at least 2, 3, 4, 5, 6, 7, 8, 9, 10, 15, 20, 25, 30, 35, 40, 45, 50, 100, 500, or 1000-fold to produce a dilution series comprising at least 2, 3, 4, 5, 6, 7, 8, 9, 10, 11, 12, 13, 14, 15, 16, 17, 18, 19, 20, 25, 30, 35, 40, 45, or 50 descending concentrations of the sample or reagent. For example, a dilution series comprising a 2-fold serial dilution of a capture antibody reagent at a1 mg/ml starting concentration can be produced by mixing an amount of the starting concentration of capture antibody with an equal amount of a dilution buffer to create a 0.5 mg/ml concentration of the capture antibody, and repeating the process to obtain capture antibody concentrations of 0.25 mg/ml, 0.125 mg/ml, 0.0625 mg/ml, 0.0325 mg/ml, and the like.

The term "superior dynamic range" as used herein refers to the ability of an assay to detect a specific analyte in as few as one cell or in as many as thousands of cells. For example, the immunoassays described herein possess superior dynamic range because they advantageously detect a particular signal transduction molecule of interest in about 1-10,000 cells (*e.g.,* about 1, 5, 10, 25, 50, 75, 100, 250, 500, 750, 1000, 2500, 5000, 7500, or 10,000 cells) using a dilution series of capture antibody concentrations.

The term "sample" as used herein includes any biological specimen obtained from a patient. Samples include, without limitation, colon polyps, rectal polyps, whole blood, plasma, serum, red blood cells, white blood cells (*e.g.,* peripheral blood mononuclear cells), ductal lavage fluid, ascites, pleural efflux, nipple aspirate, lymph (*e.g.,* disseminated tumor cells of the lymph node), bone marrow aspirate, saliva, urine, stool *(i.e.,* feces), sputum, bronchial lavage fluid, tears, fine needle aspirate (*e.g.,* harvested by random periareolar fine needle aspiration), any other bodily fluid, a tissue sample (*e.g.,* tumor tissue) such as a biopsy of a tumor (*e.g.,* needle biopsy) or a lymph node (*e.g.,* sentinel lymph node biopsy), a tissue sample (*e.g.,* tumor tissue) such as a polyptectomy or surgical resection of a tumor, and cellular extracts thereof. In some embodiments, the sample is whole blood or a fractional component thereof such as plasma, serum, or a cell pellet. In other embodiments, the sample is obtained by isolating circulating cells of a solid tumor from whole blood or a cellular fraction thereof using any technique known in the art. In yet other embodiments, the sample is a formalin fixed paraffin embedded (FFPE) tumor tissue sample, *e.g.,* from a solid tumor such as colorectal cancer. In particular embodiments, the sample is a tumor lysate or extract prepared from frozen tissue obtained from a subject having colorectal cancer. In preferred embodiments, the sample is a cell lysate from a polyp sample after polypectomy.

The term "subject" or "patient" or "individual" typically includes humans, but can also include, other animals such as, *e.g.,* other primates, rodents, canines, felines, equines, ovines, porcines, and the like.

An "array" or "microarray" comprises a distinct set and/or dilution series of capture antibodies immobilized or restrained on a solid support such as, for example, glass (*e.g.,* a glass slide), plastic, chips, pins, filters, beads (*e*.*g*., magnetic beads, polystyrene beads, *etc*.), paper, membrane (*e*.*g*., nylon, nitrocellulose, polyvinylidene fluoride (PVDF), *etc.*), fiber bundles, or any other suitable substrate. The capture antibodies are generally immobilized or restrained on the solid support via covalent or noncovalent interactions (*e.g.,* ionic bonds, hydrophobic interactions, hydrogen bonds, Van der Waals forces, dipole-dipole bonds). In certain instances, the capture antibodies comprise capture tags which interact with capture agents bound to the solid support. The arrays used in the assays described herein typically comprise a plurality of different capture antibodies and/or capture antibody concentrations that are coupled to the surface of a solid support in different known/addressable locations.

The term "capture antibody" is intended to include an immobilized antibody which is specific for (*i.e.,* binds, is bound by, or forms a complex with) one or more analytes of interest in a sample such as a cellular extract. In particular embodiments, the capture antibody is restrained on a solid support in an array. Suitable capture antibodies for immobilizing any of a variety of signal transduction molecules on a solid support are available from Upstate (Temecula, CA), Biosource (Camarillo, CA), Cell Signaling Technologies (Danvers, MA), R&D Systems (Minneapolis, MN), Lab Vision (Fremont, CA), Santa Cruz Biotechnology (Santa Cruz, CA), Sigma (St. Louis, MO), and BD Biosciences (San Jose, CA).

The term "detection antibody" as used herein includes an antibody comprising a detectable label which is specific for (*i.e.,* binds, is bound by, or forms a complex with) one or more analytes of interest in a sample. The term also encompasses an antibody which is specific for one or more analytes of interest, wherein the antibody can be bound by another species that comprises a detectable label. Examples of detectable labels include, but are not limited to, biotin/streptavidin labels, nucleic acid (*e.g.,* oligonucleotide) labels, chemically reactive labels, fluorescent labels, enzyme labels, radioactive labels, and combinations thereof. Suitable detection antibodies for detecting the activation state and/or total amount of any of a variety of signal transduction molecules are available from Upstate (Temecula, CA), Biosource (Camarillo, CA), Cell Signaling Technologies (Danvers, MA), R&D Systems (Minneapolis, MN), Lab Vision (Fremont, CA), Santa Cruz Biotechnology (Santa Cruz, CA), Sigma (St. Louis, MO), and BD Biosciences (San Jose, CA). As a non-limiting example, phospho-specific antibodies against various phosphorylated forms of signal transduction molecules such as HER1, HER2, HER3, PI3K, AKT, ERK, RSK, cMET, IGF1R, PRAS, RPS6, c-KIT, c-Src, FLK-1, PDGFRA, PDGFRB, MAPK, PTEN, Raf, and MEK are available from Santa Cruz Biotechnology.

The term "activation state-dependent antibody" includes a detection antibody which is specific for (*i.e.*, binds, is bound by, or forms a complex with) a particular activation state of one or more analytes of interest in a sample. In preferred embodiments, the activation state-dependent antibody detects the phosphorylation, ubiquitination, and/or complexation state of one or more analytes such as one or more signal transduction molecules. In some embodiments, the phosphorylation of members of the EGFR family of receptor tyrosine kinases and/or the formation of heterodimeric complexes between EGFR family members is detected using activation state-dependent antibodies. In particular embodiments, activation state-dependent antibodies are useful for detecting one or more sites of phosphorylation in one or more of the following signal transduction molecules (phosphorylation sites correspond to the position of the amino acid in the human protein sequence): EGFR/HER1/ErbB1 (*e.g.,* tyrosine (Y) 1068); ErbB2/HER2 (*e.g.,* Y1248); ErbB3/HER3 (*e.g.,* Y1289); ErbB4/HER4 (*e.g.,* Y1284); c-Met (*e.g.,* Y1003, Y1230, Y1234, Y1235, and/or Y1349); SGK3 (*e.g.,* threonine (T) 256 and/or serine (S) 422); 4E-BP1 (*e.g.,* T70); ERK1 (*e.g.,* T185, Y187, T202, and/or Y204); ERK2 (*e.g.,* T185, Y187, T202, and/or Y204); IGFR1 (*e*.*g*., Y1158, Y1161, Y1162 and/or Y1163); MEK (*e.g.,* S217 and/or S221); PIK3R1 (*e.g.,* Y688); PDK1 (*e.g.,* S241); P70S6K (*e.g.,* T229, T389, and/or S421); PTEN (*e.g.,* S380); AKT1 (*e*.*g*., S473 and/or T308); AKT2 (*e.g.,* S474 and/or T309); AKT3 (*e.g.,* S472 and/or T305); GSK-3β (*e.g.,* S9); NFKB (*e.g.,* S536); IKB (*e.g.,* S32); BAD (*e.g.,* S112 and/or S136); mTOR (*e*.*g*., S2448); Rsk-1 (*e.g.,* T357, T359, T573, S221, S380 and/or S363); JNK (*e.g.,* T183 and/or Y185); P38 (*e.g.,* T180 and/or Y182); SHC (*e.g.,* Y239, Y240, Y317, Y349, and/or Y427); STAT3 (*e*.*g*., Y705 and/or S727); CK (*e.g.,* S23, S73, S73, and/or S43 1)FAK (*e*.*g*., Y397, Y576, S722, Y861, and/or S910); RB (*e.g.,* S249, T252, S612, and/or S780); RB1 (*e.g.,* S780); adducin (*e.g.,* S662 and/or S724); PYK2 (*e.g.,* Y402 and/or Y881); PKCa (*e.g.,* S657); PKCα/β (*e.g.,* T368 and/or T641); PKC8 (*e*.*g*., T505); p53 (*e*.*g*., S392 and/or S20); CREB (*e.g.,* S133); c-Jun (*e*.*g*., S63); c-Src (*e.g*.*,* Y416); and paxillin (*e.g.,* Y31 and/or Y118).

The term "activation state-independent antibody" includes a detection antibody which is specific for (*i.e.,* binds, is bound by, or forms a complex with) one or more analytes of interest in a sample irrespective of their activation state. For example, the activation state-independent antibody can detect both phosphorylated and unphosphorylated forms of one or more analytes such as one or more signal transduction molecules.

The term "incubating" is used synonymously with "contacting" and "exposing" and does not imply any specific time or temperature requirements unless otherwise indicated.

"Receptor tyrosine kinases" or "RTKs" include a family of fifty-six (56) proteins characterized by a transmembrane domain and a tyrosine kinase motif. RTKs function in cell signaling and transmit signals regulating growth, differentiation, adhesion, migration, and apoptosis. The mutational activation and/or overexpression of receptor tyrosine kinases transforms cells and often plays a crucial role in the development of cancers. RTKs have become targets of various molecularly targeted agents such as trastuzumab, cetuximab, gefitinib, erlotinib, sunitinib, imatinib, nilotinib, and the like. One well-characterized signal transduction pathway is the MAP kinase pathway, which is responsible for transducing the signal from epidermal growth factor (EGF) to the promotion of cell proliferation in cells.

The terms "disease-free survival" and "DFS" as used herein include the length of time after treatment for a specific disease (*e.g.,* cancer) during which a patient survives with no sign of the disease (*e*.*g*., without known recurrence). In certain embodiments, disease-free survival is a clinical parameter used to evaluate the efficacy of a particular therapy, which is usually measured in units of 1 or 5 years.

The term "progression-free survival" includes the length of time during and after treatment for a specific disease (*e.g.,* cancer) in which a patient is living with the disease without additional symptoms of the disease.

The term "overall survival" includes the clinical endpoint describing patients who are alive for a defined period of time after being diagnosed with or treated for a disease, such as cancer.

### III. Description of the Embodiments

The present disclosure provides compositions and methods for detecting the status (e.g., expression and/or activation levels) of components of signal transduction pathways in pre-cancerous cells from a patient suspected of developing colorectal cancer. In certain aspects, the present disclosure also provides compositions and methods for selecting appropriate therapies to downregulate or shut down one or more deregulated signal transduction pathways. Thus, certain embodiments of the invention may be used to facilitate the design of personalized therapies based on the particular molecular signature provided by the collection of total and activated signal transduction proteins in a given patient's pre-malignant polyp sample.

In particular aspects, the present disclosure provides molecular markers (analytes) that enable the diagnosis of colorectal cancer at an early stage of the disease. In specific embodiments, measuring the level of expression and/or activation (phosphorylation) of at least one or more analytes such as HER1, HER2, HER3, cMET, PI3K, IGF1R, SHC, CK, AKT, ERK, MEK, PRSK, PRAS, RPS6 and a combination thereof, is particularly useful for predicting an individual's risk of developing colorectal cancer and/or selecting a suitable anticancer drug for the treatment thereof.

In some embodiments, the present invention provides a method for diagnosing the risk of developing colorectal cancer (CRC) in a subject, the method comprising:
(a) lysing a cell from a polyp sample taken from the subject to form a cell lysate;
(b) measuring the activation and/or (total) expression level of at least one signal transduction analyte in the cell lysate, wherein the at least one signal transduction analyte is HER1; and
(c) indicating whether the subject is at risk of developing CRC based upon the activation and/or expression level of the at least one signal transduction analyte compared to that of a control.

In some embodiments, the present invention provides a method for identifying a subject as likely to develop colorectal cancer (CRC), the method comprising:
a) measuring the activation and/or expression level of at least one signal transduction analyte in a cell lysate obtained from a polyp sample taken from the subject, wherein the at least one signal transduction analyte is HER1;
b) indicating whether the subject is likely to develop CRC based upon the activation and/or expression level of the at least one signal transduction analyte compared to that of a control; and
c) determining if the polyp sample is pre-cancerous.

In some embodiments, the present invention provides a method for identifying a subject as likely to develop colorectal cancer (CRC), the method comprising: measuring the activation and/or expression level of at least one signal transduction analyte in a cell lysate obtained from a polyp sample taken from the subject, wherein the at least one signal transduction analyte is HER1, and wherein the subject is likely to develop CRC when the measured activation and/or expression level of the at least one signal transduction analyte is higher compared to that of a control.

The present disclosure provides a method for staging colorectal cancer in a subject. In particular, the method is useful for determining that an individual has localized (Stage 0 to Stage II; T to T3) colorectal cancer wherein the cancer cells are confined within the wall of the colon.

In some aspects, the method provides a method for selecting a therapy for the treatment of a patient determined to be at risk of developing CRC. The method comprises:
(a) lysing a cell from a polyp sample taken from the subject to form a cell lysate;
(b) measuring the activation and/or (total) expression level of at least one signal transduction analyte in the cell lysate;
(c) indicating whether the subject is at risk of developing CRC based upon the activation and/or expression level of the at least one signal transduction analyte compared to that of a control; and
(d) selecting a suitable anticancer drug for the treatment of colorectal cancer based upon the activation and/or expression level of the at least one signal transduction analyte determined in step (b).

For instance, if it is determined that the subject is at high risk of developing CRC and the subject's polyp sample expresses a high level of HER2, it may be suitable to recommend the administration of an anti-HER2 therapeutic drug.

In certain embodiments, the step of measuring the activation and/or (total) expression level of at least one signal transduction analyte in the cell lysate includes measuring the activation and/or expression level of any combination of two, three, four, five, six, seven, eight, nine, ten, eleven, twelve, thirteen, or fourteen of signal transduction analytes. In some embodiments, the signal transduction analytes further include components of the HER1, HER2, HER3, IGFR1, PI3K, ERK, cMET, AKT, and/or MEK signaling transduction pathways. In other embodiments, the analytes include components of other cancer pathways activated in cancer (*e.g.,* colorectal cancer). In some embodiments, an algorithm is used to interpret the activation and/or expression level of a signal transduction analyte in a test sample relative to that of a control.

In some embodiments, if the activation and/or expression level of at least one signal transduction analyte (*e.g*., HER1, HER2, HER3, cMET, PI3K, IGF1R, SHC, CK, AKT, ERK, MEK, PRSK, PRAS and/or RPS6) in the patient sample is higher than that of a sample (*e.g*., benign polyp or mucosa, non-cancerous polyp or mucosa, or non-adenomatous polyp or mucosa) from a control, such as an individual that does not have CRC or is not at risk of having CRC, it is determined that the patient is at risk of developing CRC. In some embodiments, if the activation and/or expression level of at least one signal transduction analyte is the patient sample is substantially equal to that of a sample (*e.g.,* adenoma, or malignant polyp) from a positive control, such as an individual that has CRC, it is determined that the patient is at risk of developing CRC.

In some embodiments, if the activation and/or expression level of at least one signal transduction analyte in the sample is lower than a threshold cut-off, it is indicated that the patient is not at risk of developing CRC. In other embodiments, if the activation/and or expression level of at least one signal transduction analyte in the sample is higher than a threshold cut-off, it is indicated that the patient is at risk of developing CRC. In some aspects, the threshold cut-off value is determined using patient data sets and statistical analysis known to those skilled in the art.

The method described herein can be used to monitor CRC progression in an individual such that the activation and/or expression levels of at least one analyte are measured samples from both a first time point and a second (later) time point. An increase in the activation level and/or expression of the analyte indicates that the individual is progressing towards a late stage of CRC. The method can also be used to determine whether the individual should receive a therapeutic drug for the treatment of CRC. In some instances, the therapeutic drug is directed to one or more of the signal transduction analytes that are activated in the patient.

### IV. Sample Isolation

In some embodiments, the colorectal polyp tissue of the method described herein has been harvested from an individual by polypectomy. One skilled in the art recognizes that a polypectomy is a surgical procedure that removes colorectal polyps. Typically, a colonscope is used to locate a polyp in the individual's gastrointestinal tract and tools such as biopsy forceps, snares, detachable loop ligating devices are used to safely remove the polyp and collect it for analysis. Prior to polypectomy, the colon is cleared and completely cleaned.

In some embodiments, the polyp tissue is frozen and stored at -80°C or at liquid nitrogen prior to cell lysis.

To preserve the *in situ* activation states, signal transduction proteins (analytes) are typically extracted shortly after the tissue samples are isolated. Preferably the tissues samples are lysed within 96, 72, 48, 24, 6, or 1 hr, more preferably, within 30, 15, or 5 minutes. The isolated cells may also be incubated with growth factors usually at nanomolar to micromolar concentrations for about 1-30 minutes to resuscitate or stimulate signal transducer activation (*see, e.g.,* Irish et al., Cell, 118:217-228 (2004)). Stimulatory growth factors include epidermal growth factor (EGF), heregulin (HRG), TGF-α, PIGF, angiopoietin (Ang), NRG1, PGF, TNF-α, VEGF, PDGF, IGF, FGF, HGF, cytokines, and the like. After isolation (e.g., tissue biopsy by polypectomy), the cells are lysed to extract the signal transduction proteins using any technique known in the art. Preferably, the cell lysis is initiated between about 1-360 minutes. In some embodiments, a commercially available lysis buffer including, but not limited to, MSD lysis buffer (Meso Scale Discovery, Gaithersburg, MD) and other buffers know to those of skill in the art is used to lyse cells in the tissue (*e.g*., polyp) sample. In some instances, cells are lysed according to the manufacturer's instruction protocol. In some embodiments, the lysate is stored at -80°C until use.

### V. Analytes

Non-limiting examples of analytes such as signal transduction molecules that can be interrogated for expression (*e.g.,* total amount) levels and/or activation (*e.g.,* phosphorylation) levels in a cellular extract include receptor tyrosine kinases, non-receptor tyrosine kinases, tyrosine kinase signaling cascade components, nuclear hormone receptors, nuclear receptor coactivators, nuclear receptor repressors, and combinations thereof.

In one embodiment, the methods of the present invention comprise determining the expression level (*e.g.,* total amount) and/or activation level (*e.g*., level of phosphorylation or complex formation) of at least one or more of the following analytes in a cellular extract: (1) HER1/EGFR/ErbB1; (2) HER2/ErbB2; (3) p95HER2 (truncated HER2); (4) HER3/ErbB3; (5) c-MET; (6) IGF1R; (7) CK; (8) PI3K (*e.g.,* PIK3CA and/or PIK3R1); (9) SHC; (10) AKT; (11) PRAS; (12) RSK; (13) ERK (*e.g.,* Erk1 (MAPK3) and/or Erk2 (MAPK1)); (14) MEK and a combination thereof. With the proviso that at least HER1 is determined.

In one particular embodiment, the present invention comprises determining the expression level (*e.g.,* total amount) and/or activation level (*e.g.,* level of phosphorylation or complex formation) of at least one, two, three, four, five, six, seven, eight, nine, or ten of the following analytes: HER1, HER2, HER3, cMET, IGF-1R, PI3K (*e.g.,* PIK3CA and/or PIK3R1), AKT, ERK (*e.g*., ERK1 (MAPK3) and/or ERK2 (MAPK1)), MEK, RSK, PRAS, RPS6, CK, and SHC. With the proviso that at least HER1 is determined.

In some embodiments, the activation level corresponds to a level of phosphorylation of HER1, HER2, HER3, cMET, PI3K, IGF-1R, SHC, CK, AKT, ERK, MEK, RSK, PRAS, RPS6 and a combination thereof. With the proviso that at least HER1 is determined. In certain other embodiments, the activation level further includes a level of a PI3K complex. Examples of PI3K complexes include, without limitation, one or more complexes comprising a dimerized receptor tyrosine kinase pair, a PI3K p85 subunit (*e.g.,* PIK3R1), and a PI3K p110 subunit (*e.g.,* an α or β subunit such as PIK3CA or PIK3CB); *see*, for example, International Patent Publication No. WO2013/033623.

In certain embodiments, the present invention further comprises determining the expression level (*e.g.,* total amount) and/or activation level (*e.g.,* level of phosphorylation or complex formation) of one or more (*e.g.,* at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more) additional analytes in a cellular extract. In some embodiments, the one or more (*e.g.,* at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more) additional analytes comprises one or more signal transduction molecules selected from the group consisting of receptor tyrosine kinases, non-receptor tyrosine kinases, tyrosine kinase signaling cascade components, nuclear hormone receptors, nuclear receptor coactivators, nuclear receptor repressors, and combinations thereof.

In particular embodiments, the present invention further comprises determining the expression level (*e.g*., total amount) and/or activation level (*e.g.,* level of phosphorylation or complex formation) of one or any combination of 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more of the following additional analytes in a cellular extract: HER4, MEK, PTEN, SGK3, 4E-BP1, PDK1, PDK2, GSK-3β, Raf, SRC, NFkB-IkB, mTOR, EPH-A, EPH-B, EPH-C, EPH-D, FLT-3, TIE-1, TIE-2, c-FMS, Abl, FTL 3, RET, FGFR1, FGFR2, FGFR3, FGFR4, ER, PR, NCOR, AIB1, RON, PIP2, PIP3, p27, protein tyrosine phosphatases (*e.g*., PTP1B, PTPN13, BDP1, *etc*.), receptor dimers, and combinations thereof.

Total expression and activation (*e.g.,* phosphorylation) levels of analytes such as signal transduction molecules can be determined using any of a variety of techniques. In certain embodiments, the expression and/or activation (*e.g.,* phosphorylation) level and/or status of analytes such as signal transduction molecules in samples such as cellular extracts of cancer cells is detected with an immunoassay such as a single detection assay or a proximity dual detection assay (*e.g*., CEER™) as described herein.

### VI. Single Detection Assays

In some embodiments, the assay for detecting the expression and/or activation level of one or more analytes of interest in a cellular extract of cells such as tumor cells is a multiplex, high-throughput two-antibody assay having superior dynamic range. As a non-limiting example, the two antibodies used in the assay can comprise: (1) a capture antibody specific for a particular analyte of interest; and (2) a detection antibody specific for an activated form of the analyte (*i.e.,* activation state-dependent antibody). The activation state-dependent antibody is capable of detecting, for example, the phosphorylation, ubiquitination, and/or complexation state of the analyte. Alternatively, the detection antibody comprises an activation state-independent antibody, which detects the total amount of the analyte in the cellular extract. The activation state-independent antibody is generally capable of detecting both the activated and non-activated forms of the analyte.

In one particular embodiment, the two-antibody assay for detecting the expression or activation level of an analyte of interest comprises:
(i) incubating the cellular extract with one or a plurality of dilution series of capture antibodies to form a plurality of captured analytes;
(ii) incubating the plurality of captured analytes with detection antibodies specific for the corresponding analytes to form a plurality of detectable captured analytes, wherein the detection antibodies comprise activation state-dependent antibodies for detecting the activation (*e.g*., phosphorylation) level of the analyte or activation state-independent antibodies for detecting the expression level (*e.g.,* total amount) of the analyte;
(iii) incubating the plurality of detectable captured analytes with first and second members of a signal amplification pair to generate an amplified signal; and
(iv) detecting the amplified signal generated from the first and second members of the signal amplification pair.

The two-antibody assays described herein are typically antibody-based arrays which comprise a plurality of different capture antibodies at a range of capture antibody concentrations that are coupled to the surface of a solid support in different addressable locations. Examples of suitable solid supports for use in the present invention are described above.

The capture antibodies and detection antibodies are preferably selected to minimize competition between them with respect to analyte binding (*i.e.,* both capture and detection antibodies can simultaneously bind their corresponding signal transduction molecules).

In one embodiment, the detection antibodies comprise a first member of a binding pair (*e.g.,* biotin) and the first member of the signal amplification pair comprises a second member of the binding pair (*e.g.,* streptavidin). The binding pair members can be coupled directly or indirectly to the detection antibodies or to the first member of the signal amplification pair using methods well-known in the art. In certain instances, the first member of the signal amplification pair is a peroxidase (*e.g.,* horseradish peroxidase (HRP), catalase, chloroperoxidase, cytochrome c peroxidase, eosinophil peroxidase, glutathione peroxidase, lactoperoxidase, myeloperoxidase, thyroid peroxidase, deiodinase, *etc.*), and the second member of the signal amplification pair is a tyramide reagent (*e.g.,* biotin-tyramide). In these instances, the amplified signal is generated by peroxidase oxidization of the tyramide reagent to produce an activated tyramide in the presence of hydrogen peroxide (H₂O₂).

The activated tyramide is either directly detected or detected upon the addition of a signal-detecting reagent such as, for example, a streptavidin-labeled fluorophore or a combination of a streptavidin-labeled peroxidase and a chromogenic reagent. Examples of fluorophores suitable for use in the present invention include, but are not limited to, an Alexa Fluor® dye (*e.g*., Alexa Fluor® 555), fluorescein, fluorescein isothiocyanate (FITC), Oregon Green™; rhodamine, Texas red, tetrarhodamine isothiocynate (TRITC), a CyDye™ fluor (*e.g.,* Cy2, Cy3, Cy5), and the like. The streptavidin label can be coupled directly or indirectly to the fluorophore or peroxidase using methods well-known in the art. Non-limiting examples of chromogenic reagents suitable for use in the present invention include 3,3',5,5'-tetramethylbenzidine (TMB), 3,3'-diaminobenzidine (DAB), 2,2'-azino-bis(3-ethylbenzothiazoline-6-sulfonic acid) (ABTS), 4-chloro-1-napthol (4CN), and/or porphyrinogen.

An exemplary protocol for performing the two-antibody assays described herein is provided in U.S. Patent No. 8,163,499.

In another embodiment of a two-antibody approach, the present invention provides a method for detecting the expression or activation level of a truncated receptor, the method comprising:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with a dilution series of one or a plurality of capture antibodies specific for an intracellular domain (ICD) binding region of the full-length receptor to form a plurality of captured truncated receptors;
(iv) incubating the plurality of captured truncated receptors with detection antibodies specific for an ICD binding region of the full-length receptor to form a plurality of detectable captured truncated receptors, wherein the detection antibodies comprise activation state-dependent antibodies for detecting the activation (*e.g.,* phosphorylation) level of the truncated receptor or activation state-independent antibodies for detecting the expression level (*e.g*., total amount) of the truncated receptor;
(v) incubating the plurality of detectable captured truncated receptors with first and second members of a signal amplification pair to generate an amplified signal; and
(vi) detecting an amplified signal generated from the first and second members of the signal amplification pair.

In certain embodiments, the truncated receptor is p95HER2 and the full-length receptor is HER2. In certain other embodiments, the plurality of beads specific for an extracellular domain (ECD) binding region comprises a streptavidin-biotin pair, wherein the biotin is attached to the bead and the biotin is attached to an antibody (*e.g.,* wherein the antibody is specific for the ECD binding region of the full-length receptor).

U.S. Patent No. 8,163,499, shows that beads coated with an antibody directed to the extracellular domain (ECD) of a receptor of interest binds the full-length receptor (*e.g.,* HER2), but not the truncated receptor (*e.g.,* p95HER2) to remove any full-length receptor from the assay. U.S. Patent No. 8,163,499 shows that the truncated receptor (*e.g*., p95HER2), once bound to a capture antibody, may then be detected by a detection antibody that is specific for the intracellular domain (ICD) of the full-length receptor (*e.g.,* HER2). The detection antibody may be directly conjugated to horseradish peroxidase (HRP). Tyramide signal amplification (TSA) may then be performed to generate a signal to be detected. The expression level or activation state of the truncated receptor (*e.g*., p95HBR2) can be interrogated to determine, *e.g.,* its total concentration or its phosphorylation state, ubiquitination state, and/or complexation state.

The present disclosure provides kits for performing the two-antibody assays described above comprising: (a) a dilution series of one or a plurality of capture antibodies restrained on a solid support; and (b) one or a plurality of detection antibodies (*e.g.,* activation state-independent antibodies and/or activation state-dependent antibodies). In some instances, the kits can further contain instructions for methods of using the kit to detect the expression levels and/or activation states of one or a plurality of signal transduction molecules of cells such as tumor cells. The kits may also contain any of the additional reagents described above with respect to performing the specific methods of the present invention such as, for example, first and second members of the signal amplification pair, tyramide signal amplification reagents, wash buffers, *etc.*

### VII. Proximity Dual Detection Assays

In some embodiments, the assay for detecting the expression and/or activation level of one or more analytes of interest in a cellular extract of polyp cells is a multiplex, high-throughput proximity (*i.e.,* three-antibody) assay having superior dynamic range. As a non-limiting example, the three antibodies used in the proximity assay can comprise: (1) a capture antibody specific for a particular analyte of interest; (2) a detection antibody specific for an activated form of the analyte (*i.e.,* activation state-dependent antibody); and (3) a detection antibody which detects the total amount of the analyte (*i.e.,* activation state-independent antibody). The activation state-dependent antibody is capable of detecting, *e.g.,* the phosphorylation, ubiquitination, and/or complexation state of the analyte, while the activation state-independent antibody is capable of detecting the total amount (*i.e.,* both the activated and non-activated forms) of the analyte.

In one particular embodiment, the proximity assay for detecting the activation level or status of an analyte of interest comprises:
(i) incubating the cellular extract with one or a plurality of dilution series of capture antibodies to form a plurality of captured analytes;
(ii) incubating the plurality of captured analytes with detection antibodies comprising one or a plurality of activation state-independent antibodies and one or a plurality of activation state-dependent antibodies specific for the corresponding analytes to form a plurality of detectable captured analytes,
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(iii) incubating the plurality of detectable captured analytes with a second member of the signal amplification pair to generate an amplified signal; and
(iv) detecting the amplified signal generated from the first and second members of the signal amplification pair.

In another particular embodiment, the proximity assay for detecting the activation level or status of an analyte of interest that is a truncated receptor comprises:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with one or a plurality of capture antibodies specific for an intracellular domain (ICD) binding region of the full-length receptor to form a plurality of captured truncated receptors;
(iv) incubating the plurality of captured truncated receptors with detection antibodies comprising one or a plurality of activation state-independent antibodies and one or a plurality of activation state-dependent antibodies specific for an ICD binding region of the full-length receptor to form a plurality of detectable captured truncated receptors,
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(v) incubating the plurality of detectable captured truncated receptors with a second member of the signal amplification pair to generate an amplified signal; and
(vi) detecting the amplified signal generated from the first and second members of the signal amplification pair.

In certain embodiments, the truncated receptor is p95HER2 and the full-length receptor is HER2. In certain other embodiments, the plurality of beads specific for an extracellular domain (ECD) binding region comprises a streptavidin-biotin pair, wherein the biotin is attached to the bead and the biotin is attached to an antibody (*e.g.,* wherein the antibody is specific for the ECD binding region of the full-length receptor).

In alternative embodiments, the activation state-dependent antibodies can be labeled with a facilitating moiety and the activation state-independent antibodies can be labeled with a first member of a signal amplification pair.

As another non-limiting example, the three antibodies used in the proximity assay can comprise: (1) a capture antibody specific for a particular analyte of interest; (2) a first detection antibody which detects the total amount of the analyte (*i.e.,* a first activation state-independent antibody); and (3) a second detection antibody which detects the total amount of the analyte (*i.e.,* a second activation state-independent antibody). In preferred embodiments, the first and second activation state-independent antibodies recognize different (*e.g*., distinct) epitopes on the analyte.

In one particular embodiment, the proximity assay for detecting the expression level of an analyte of interest comprises:
(i) incubating the cellular extract with one or a plurality of dilution series of capture antibodies to form a plurality of captured analytes;
(ii) incubating the plurality of captured analytes with detection antibodies comprising one or a plurality of first and second activation state-independent antibodies specific for the corresponding analytes to form a plurality of detectable captured analytes,
   wherein the first activation state-independent antibodies are labeled with a facilitating moiety, the second activation state-independent antibodies are labeled with a first member of a signal amplification pair, and the facilitating moiety generates an oxidizing agent which channels to and reacts with the first member of the signal amplification pair;
(iii) incubating the plurality of detectable captured analytes with a second member of the signal amplification pair to generate an amplified signal; and
(iv) detecting the amplified signal generated from the first and second members of the signal amplification pair.

In another particular embodiment, the proximity assay for detecting the expression level of an analyte of interest that is a truncated receptor comprises:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with one or a plurality of capture antibodies specific for an intracellular domain (ICD) binding region of the full-length receptor to form a plurality of captured truncated receptors;
(iv) incubating the plurality of captured truncated receptors with detection antibodies comprising one or a plurality of first and second activation state-independent antibodies specific for an ICD binding region of the full-length receptor to form a plurality of detectable captured truncated receptors,
   wherein the first activation state-independent antibodies are labeled with a facilitating moiety, the second activation state-independent antibodies are labeled with a first member of a signal amplification pair, and the facilitating moiety generates an oxidizing agent which channels to and reacts with the first member of the signal amplification pair;
(v) incubating the plurality of detectable captured truncated receptors with a second member of the signal amplification pair to generate an amplified signal; and
(vi) detecting the amplified signal generated from the first and second members of the signal amplification pair.

In certain embodiments, the truncated receptor is p95HER2 and the full-length receptor is HER2. In certain other embodiments, the plurality of beads specific for an extracellular domain (ECD) binding region comprises a streptavidin-biotin pair, wherein the biotin is attached to the bead and the biotin is attached to an antibody (*e.g*, wherein the antibody is specific for the ECD binding region of the full-length receptor).

In alternative embodiments, the first activation state-independent antibodies can be labeled with a first member of a signal amplification pair and the second activation state-independent antibodies can be labeled with a facilitating moiety.

The proximity assays described herein are typically antibody-based arrays which comprise one or a plurality of different capture antibodies at a range of capture antibody concentrations that are coupled to the surface of a solid support in different addressable locations. Examples of suitable solid supports for use in the present invention are described above.

The capture antibodies, activation state-independent antibodies, and activation state-dependent antibodies are preferably selected to minimize competition between them with respect to analyte binding (*i.e.,* all antibodies can simultaneously bind their corresponding signal transduction molecules).

In some embodiments, activation state-independent antibodies for detecting activation levels of one or more of the analytes or, alternatively, first activation state-independent antibodies for detecting expression levels of one or more of the analytes further comprise a detectable moiety. In such instances, the amount of the detectable moiety is correlative to the amount of one or more of the analytes in the cellular extract. Examples of detectable moieties include, but are not limited to, fluorescent labels, chemically reactive labels, enzyme labels, radioactive labels, and the like. Preferably, the detectable moiety is a fluorophore such as an Alexa Fluor® dye (*e.g*., Alexa Fluor® 647), fluorescein, fluorescein isothiocyanate (FITC), Oregon Green™; rhodamine, Texas red, tetrarhodamine isothiocynate (TRITC), a CyDye™ fluor (*e.g.,* Cy2, Cy3, Cy5), and the like. The detectable moiety can be coupled directly or indirectly to the activation state-independent antibodies using methods well-known in the art.

In certain instances, activation state-independent antibodies for detecting activation levels of one or more of the analytes or, alternatively, first activation state-independent antibodies for detecting expression levels of one or more of the analytes are directly labeled with the facilitating moiety. The facilitating moiety can be coupled to activation state-independent antibodies using methods well-known in the art. A suitable facilitating moiety for use in the present invention includes any molecule capable of generating an oxidizing agent which channels to (*i.e.,* is directed to) and reacts with (*i.e.,* binds, is bound by, or forms a complex with) another molecule in proximity (*i.e.,* spatially near or close) to the facilitating moiety. Examples of facilitating moieties include, without limitation, enzymes such as glucose oxidase or any other enzyme that catalyzes an oxidation/reduction reaction involving molecular oxygen (O₂) as the electron acceptor, and photosensitizers such as methylene blue, rose bengal, porphyrins, squarate dyes, phthalocyanines, and the like. Non-limiting examples of oxidizing agents include hydrogen peroxide (H₂O₂), a singlet oxygen, and any other compound that transfers oxygen atoms or gains electrons in an oxidation/reduction reaction. Preferably, in the presence of a suitable substrate (*e.g*., glucose, light, *etc.*), the facilitating moiety (*e.g*., glucose oxidase, photosensitizer, *etc*.) generates an oxidizing agent (*e.g.,* hydrogen peroxide (H₂O₂), single oxygen, *etc*.) which channels to and reacts with the first member of the signal amplification pair (*e.g*., horseradish peroxidase (HRP), hapten protected by a protecting group, an enzyme inactivated by thioether linkage to an enzyme inhibitor, *etc*.) when the two moieties are in proximity to each other.

In certain other instances, activation state-independent antibodies for detecting activation levels of one or more of the analytes or, alternatively, first activation state-independent antibodies for detecting expression levels of one or more of the analytes are indirectly labeled with the facilitating moiety via hybridization between an oligonucleotide linker conjugated to the activation state-independent antibodies and a complementary oligonucleotide linker conjugated to the facilitating moiety. The oligonucleotide linkers can be coupled to the facilitating moiety or to the activation state-independent antibodies using methods well-known in the art. In some embodiments, the oligonucleotide linker conjugated to the facilitating moiety has 100% complementarity to the oligonucleotide linker conjugated to the activation state-independent antibodies. In other embodiments, the oligonucleotide linker pair comprises at least one, two, three, four, five, six, or more mismatch regions, *e.g*., upon hybridization under stringent hybridization conditions. One skilled in the art will appreciate that activation state-independent antibodies specific for different analytes can either be conjugated to the same oligonucleotide linker or to different oligonucleotide linkers.

The length of the oligonucleotide linkers that are conjugated to the facilitating moiety or to the activation state-independent antibodies can vary. In general, the linker sequence can be at least about 5, 10, 15, 20, 25, 30, 35, 40, 45, 50, 75, or 100 nucleotides in length. Typically, random nucleic acid sequences are generated for coupling. As a non-limiting example, a library of oligonucleotide linkers can be designed to have three distinct contiguous domains: a spacer domain; signature domain; and conjugation domain. Preferably, the oligonucleotide linkers are designed for efficient coupling without destroying the function of the facilitating moiety or activation state-independent antibodies to which they are conjugated.

The oligonucleotide linker sequences can be designed to prevent or minimize any secondary structure formation under a variety of assay conditions. Melting temperatures are typically carefully monitored for each segment within the linker to allow their participation in the overall assay procedures. Generally, the range of melting temperatures of the segment of the linker sequence is between 1-10°C. Computer algorithms (*e.g.,* OLIGO 6.0) for determining the melting temperature, secondary structure, and hairpin structure under defined ionic concentrations can be used to analyze each of the three different domains within each linker. The overall combined sequences can also be analyzed for their structural characterization and their comparability to other conjugated oligonucleotide linker sequences, *e.g.,* whether they will hybridize under stringent hybridization conditions to a complementary oligonucleotide linker.

The spacer region of the oligonucleotide linker provides adequate separation of the conjugation domain from the oligonucleotide crosslinking site. The conjugation domain functions to link molecules labeled with a complementary oligonucleotide linker sequence to the conjugation domain via nucleic acid hybridization. The nucleic acid-mediated hybridization can be performed either before or after antibody-analyte (*i.e.,* antigen) complex formation, providing a more flexible assay format. Unlike many direct antibody conjugation methods, linking relatively small oligonucleotides to antibodies or other molecules has minimal impact on the specific affinity of antibodies towards their target analyte or on the function of the conjugated molecules.

In some embodiments, the signature sequence domain of the oligonucleotide linker can be used in complex multiplexed protein assays. Multiple antibodies can be conjugated with oligonucleotide linkers with different signature sequences. In multiplex immunoassays, reporter oligonucleotide sequences labeled with appropriate probes can be used to detect cross-reactivity between antibodies and their antigens in the multiplex assay format.

Oligonucleotide linkers can be conjugated to antibodies or other molecules using several different methods. For example, oligonucleotide linkers can be synthesized with a thiol group on either the 5' or 3' end. The thiol group can be deprotected using reducing agents (*e.g*., TCEP-HCl) and the resulting linkers can be purified by using a desalting spin column. The resulting deprotected oligonucleotide linkers can be conjugated to the primary amines of antibodies or other types of proteins using heterobifunctional cross linkers such as SMCC. Alternatively, 5'-phosphate groups on oligonucleotides can be treated with watersoluble carbodiimide EDC to form phosphate esters and subsequently coupled to amine-containing molecules. In certain instances, the diol on the 3'-ribose residue can be oxidized to aldehyde groups and then conjugated to the amine groups of antibodies or other types of proteins using reductive amination. In certain other instances, the oligonucleotide linker can be synthesized with a biotin modification on either the 3' or 5' end and conjugated to streptavidin-labeled molecules.

Oligonucleotide linkers can be synthesized using any of a variety of techniques known in the art, such as those described in Usman et al., J. Am. Chem. Soc., 109:7845 (1987); Scaringe et al., Nucl. Acids Res., 18:5433 (1990); Wincott et al., Nucl. Acids Res., 23:2677-2684 (1995); and Wincott et al., Methods Mol. Bio., 74:59 (1997). In general, the synthesis of oligonucleotides makes use of common nucleic acid protecting and coupling groups, such as dimethoxytrityl at the 5'-end and phosphoramidites at the 3'-end. Suitable reagents for oligonucleotide synthesis, methods for nucleic acid deprotection, and methods for nucleic acid purification are known to those of skill in the art.

In certain instances, activation state-dependent antibodies for detecting activation levels of one or more of the analytes or, alternatively, second activation state-independent antibodies for detecting expression levels of one or more of the analytes are directly labeled with the first member of the signal amplification pair. The signal amplification pair member can be coupled to activation state-dependent antibodies to detect activation levels or second activation state-independent antibodies to detect expression levels using methods well-known in the art. In certain other instances, activation state-dependent antibodies or second activation state-independent antibodies are indirectly labeled with the first member of the signal amplification pair via binding between a first member of a binding pair conjugated to the activation state-dependent antibodies or second activation state-independent antibodies and a second member of the binding pair conjugated to the first member of the signal amplification pair. The binding pair members (*e.g.*, biotin/streptavidin) can be coupled to the signal amplification pair member or to the activation state-dependent antibodies or second activation state-independent antibodies using methods well-known in the art. Examples of signal amplification pair members include, but are not limited to, peroxidases such horseradish peroxidase (HRP), catalase, chloroperoxidase, cytochrome c peroxidase, eosinophil peroxidase, glutathione peroxidase, lactoperoxidase, myeloperoxidase, thyroid peroxidase, deiodinase, and the like. Other examples of signal amplification pair members include haptens protected by a protecting group and enzymes inactivated by thioether linkage to an enzyme inhibitor.

In one example of proximity channeling, the facilitating moiety is glucose oxidase (GO) and the first member of the signal amplification pair is horseradish peroxidase (HRP). When the GO is contacted with a substrate such as glucose, it generates an oxidizing agent (*i.e.,* hydrogen peroxide (H₂O₂)). If the HRP is within channeling proximity to the GO, the H₂O₂ generated by the GO is channeled to and complexes with the HRP to form an HRP-H₂O₂ complex, which, in the presence of the second member of the signal amplification pair (*e.g.,* a chemiluminescent substrate such as luminol or isoluminol or a fluorogenic substrate such as tyramide (*e.g.,* biotin-tyramide), homovanillic acid, or 4-hydroxyphenyl acetic acid), generates an amplified signal. Methods of using GO and HRP in a proximity assay are described in, *e.g.,* Langry et al., U.S. Dept. of Energy Report No. UCRL-ID-136797 (1999). When biotin-tyramide is used as the second member of the signal amplification pair, the HRP-H₂O₂ complex oxidizes the tyramide to generate a reactive tyramide radical that covalently binds nearby nucleophilic residues. The activated tyramide is either directly detected or detected upon the addition of a signal-detecting reagent such as, for example, a streptavidin-labeled fluorophore or a combination of a streptavidin-labeled peroxidase and a chromogenic reagent. Examples of fluorophores suitable for use in the present invention include, but are not limited to, an Alexa Fluor® dye (*e.g.,* Alexa Fluor® 555), fluorescein, fluorescein isothiocyanate (FITC), Oregon Green™; rhodamine, Texas red, tetrarhodamine isothiocynate (TRITC), a CyDye™ fluor (*e.g.,* Cy2, Cy3, Cy5), and the like. The streptavidin label can be coupled directly or indirectly to the fluorophore or peroxidase using methods well-known in the art. Non-limiting examples of chromogenic reagents suitable for use in the present invention include 3,3',5,5'-tetramethylbenzidine (TMB), 3,3'-diaminobenzidine (DAB), 2,2'-azino-bis(3-ethylbenzothiazoline-6-sulfonic acid) (ABTS), 4-chloro-1-napthol (4CN), and/or porphyrinogen.

In another example of proximity channeling, the facilitating moiety is a photosensitizer and the first member of the signal amplification pair is a large molecule labeled with multiple haptens that are protected with protecting groups that prevent binding of the haptens to a specific binding partner (*e.g.,* ligand, antibody, *etc*.). For example, the signal amplification pair member can be a dextran molecule labeled with protected biotin, coumarin, and/or fluorescein molecules. Suitable protecting groups include, but are not limited to, phenoxy-, analino-, olefin-, thioether-, and selenoether-protecting groups. Additional photosensitizers and protected hapten molecules suitable for use in the proximity assays of the present invention are described in U.S. Patent No. 5,807,675. When the photosensitizer is excited with light, it generates an oxidizing agent (*i.e.,* singlet oxygen). If the hapten molecules are within channeling proximity to the photosensitizer, the singlet oxygen generated by the photosensitizer is channeled to and reacts with thioethers on the protecting groups of the haptens to yield carbonyl groups (ketones or aldehydes) and sulphinic acid, releasing the protecting groups from the haptens. The unprotected haptens are then available to specifically bind to the second member of the signal amplification pair (*e.g*., a specific binding partner that can generate a detectable signal). For example, when the hapten is biotin, the specific binding partner can be an enzyme-labeled streptavidin. Exemplary enzymes include alkaline phosphatase, β-galactosidase, HRP, *etc.* After washing to remove unbound reagents, the detectable signal can be generated by adding a detectable (*e.g*., fluorescent, chemiluminescent, chromogenic, *etc*.) substrate of the enzyme and detected using suitable methods and instrumentation known in the art. Alternatively, the detectable signal can be amplified using tyramide signal amplification and the activated tyramide either directly detected or detected upon the addition of a signal-detecting reagent as described above.

In yet another example of proximity channeling, the facilitating moiety is a photosensitizer and the first member of the signal amplification pair is an enzyme-inhibitor complex. The enzyme and inhibitor (*e.g*., phosphonic acid-labeled dextran) are linked together by a cleavable linker (*e.g.,* thioether). When the photosensitizer is excited with light, it generates an oxidizing agent (*i.e.,* singlet oxygen). If the enzyme-inhibitor complex is within channeling proximity to the photosensitizer, the singlet oxygen generated by the photosensitizer is channeled to and reacts with the cleavable linker, releasing the inhibitor from the enzyme, thereby activating the enzyme. An enzyme substrate is added to generate a detectable signal, or alternatively, an amplification reagent is added to generate an amplified signal.

In a further example of proximity channeling, the facilitating moiety is HRP, the first member of the signal amplification pair is a protected hapten or an enzyme-inhibitor complex as described above, and the protecting groups comprise p-alkoxy phenol. The addition of phenylenediamine and H₂O₂ generates a reactive phenylene diimine which channels to the protected hapten or the enzyme-inhibitor complex and reacts with p-alkoxy phenol protecting groups to yield exposed haptens or a reactive enzyme. The amplified signal is generated and detected as described above (*see, e.g.,* U.S. Patent Nos. 5,532,138 and 5,445,944).

An exemplary protocol for performing the proximity assays described herein is provided in U.S. Patent No. 8,163,499.

The present disclosure provides kits for performing the proximity assays described above comprising: (a) a dilution series of one or a plurality of capture antibodies restrained on a solid support; and (b) one or a plurality of detection antibodies (*e.g*., a combination of activation state-independent antibodies and activation state-dependent antibodies for detecting activation levels and/or a combination of first and second activation state-independent antibodies for detecting expression levels). In some instances, the kits can further contain instructions for methods of using the kit to detect the expression and/or activation status of one or a plurality of signal transduction molecules of cells such as tumor cells. The kits may also contain any of the additional reagents described above with respect to performing the specific methods of the present invention such as, for example, first and second members of the signal amplification pair, tyramide signal amplification reagents, substrates for the facilitating moiety, wash buffers, *etc.*

In certain embodiments, determining the activation (*e.g.,* phosphorylation) level of the one or more analytes comprises:
(i) incubating (*e.g*., contacting) a cellular extract produced from the cell with a dilution series of capture antibodies (*e.g.,* capture antibodies specific for one or more analytes) to form a plurality of captured analytes, wherein the capture antibodies are restrained on a solid support (*e.g.,* to transform the analytes present in the cellular extract into complexes of captured analytes comprising the analytes and capture antibodies);
(ii) incubating (*e.g.,* contacting) the plurality of captured analytes with detection antibodies comprising activation state-independent antibodies specific for the corresponding analytes (*e.g*., activation state-independent antibodies specific for the one or more analytes) and activation state-dependent antibodies specific for the corresponding analytes (*e.g.,* activation state-dependent antibodies specific for the one or more analytes) to form a plurality of detectable captured analytes (*e.g*., to transform the complexes of captured analytes into complexes of detectable captured analytes comprising the captured analytes and detection antibodies),
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(iii) incubating (*e.g.,* contacting) the plurality of detectable captured analytes with a second member of the signal amplification pair to generate an amplified signal; and
(iv) detecting the amplified signal generated from the first and second members of the signal amplification pair.

In certain other embodiments, determining the activation (*e.g.,* phosphorylation) level of the one or more analytes that are truncated receptors (*e.g*., p95HER2) comprises:
(i) incubating (*e.g.,* contacting) a cellular extract produced from the cell with a plurality of beads specific for an extracellular domain (ECD) binding region of a full-length receptor (*e.g.,* full-length HER2);
(ii) removing the plurality of beads from the cellular extract, thereby removing the full-length receptor (*e.g.,* full-length HER2) to form a cellular extract devoid of the full-length receptor (*e.g.,* full-length HER2) (*e.g.,* to transform the cellular extract into a cellular extract devoid of a specific full-length receptor or family of full-length receptors);
(iii) incubating (*e.g.,* contacting) the cellular extract devoid of the full-length receptor (*e.g.,* full-length HER2) with a plurality of capture antibodies specific for an intracellular domain (ICD) binding region of the full-length receptor (*e.g.,* full-length HER2) to form a plurality of captured truncated receptors, wherein the capture antibodies are restrained on a solid support (*e.g*., to transform the truncated receptors present in a full-length receptor-depleted cellular extract into complexes of truncated receptors and capture antibodies);
(iv) incubating (*e.g.,* contacting) the plurality of captured truncated receptors with detection antibodies comprising activation state-independent antibodies and activation state-dependent antibodies specific for an ICD binding region of the full-length receptor (*e.g.,* full-length HER2) to form a plurality of detectable captured truncated receptors (*e.g.,* to transform the complexes of captured truncated receptors into complexes of detectable captured truncated receptors comprising the captured truncated receptors and detection antibodies),
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(v) incubating (*e.g.,* contacting) the plurality of detectable captured truncated receptors with a second member of the signal amplification pair to generate an amplified signal; and
(vi) detecting the amplified signal generated from the first and second members of the signal amplification pair.

The activation state-independent antibodies may be directly labeled with the facilitating moiety or indirectly labeled with the facilitating moiety, *e.g*., via hybridization between an oligonucleotide conjugated to the activation state-independent antibodies and a complementary oligonucleotide conjugated to the facilitating moiety. Similarly, the activation state-dependent antibodies may be directly labeled with the first member of the signal amplification pair or indirectly labeled with the first member of the signal amplification pair, *e.g.,* via binding between a first member of a binding pair conjugated to the activation state-dependent antibodies and a second member of the binding pair conjugated to the first member of the signal amplification pair. In certain instances, the first member of the binding pair is biotin and the second member of the binding pair is an avidin such as streptavidin or neutravidin.

In some embodiments, the facilitating moiety may be, for example, glucose oxidase. In certain instances, the glucose oxidase and the activation state-independent antibodies can be conjugated to a sulfhydryl-activated dextran molecule as described in, *e.g.,* U.S. Patent No. 8,163,499. The sulfhydryl-activated dextran molecule typically has a molecular weight of about 500kDa (*e.g.,* about 250, 300, 350, 400, 450, 500, 550, 600, 650, 700, or 750kDa). In other embodiments, the oxidizing agent may be, for example, hydrogen peroxide (H₂O₂). In yet other embodiments, the first member of the signal amplification pair may be, for example, a peroxidase such as horseradish peroxidase (HRP). In further embodiments, the second member of the signal amplification pair may be, for example, a tyramide reagent (*e.g.,* biotin-tyramide). Preferably, the amplified signal is generated by peroxidase oxidization of biotin-tyramide to produce an activated tyramide (*e.g.,* to transform the biotin-tyramide into an activated tyramide). The activated tyramide may be directly detected or indirectly detected, *e.g.,* upon the addition of a signal-detecting reagent. Non-limiting examples of signal-detecting reagents include streptavidin-labeled fluorophores and combinations of streptavidin-labeled peroxidases and chromogenic reagents such as, *e.g*., 3,3',5,5'-tetramethylbenzidine (TMB).

In certain instances, the horseradish peroxidase and the activation state-dependent antibodies can be conjugated to a sulfhydryl-activated dextran molecule. The sulfhydryl-activated dextran molecule typically has a molecular weight of about 70kDa (*e.g.,* about 40, 45, 50, 55, 60, 65, 70, 75, 80, 85, 90, 95, or 100kDa).

The truncated receptor is typically a fragment of the full-length receptor and shares an intracellular domain (ICD) binding region with the full-length receptor. In certain embodiments, the full-length receptor comprises an extracellular domain (ECD) binding region, a transmembrane domain, and an intracellular domain (ICD) binding region. Without being bound to any particular theory, the truncated receptor may arise through the proteolytic processing of the ECD of the full-length receptor or by alternative initiation of translation from methionine residues that are located before, within, or after the transmembrane domain, *e.g.,* to create a truncated receptor with a shortened ECD or a truncated receptor comprising a membrane-associated or cytosolic ICD fragment.

In certain preferred embodiments, the truncated receptor is p95HER2 and the corresponding full-length receptor is HER2 However, one skilled in the art will appreciate that the methods described herein for detecting truncated proteins can be applied to a number of different proteins including, but not limited to, the EGFR VIII mutant (implicated in glioblastoma, colorectal cancer, *etc*.), other truncated receptor tyrosine kinases, caspases, and the like. PCT Publication No. WO2009/108637, the disclosure of which is herein incorporated by reference in its entirety for all purposes, provides an exemplary embodiment of the assay methods of the present invention for detecting truncated receptors such as p95HER2 in cells using a multiplex, high-throughput, proximity dual detection microarray ELISA having superior dynamic range.

In some embodiments, the plurality of beads specific for an ECD binding region comprises a streptavidin-biotin pair, wherein the streptavidin is attached to the bead and the biotin is attached to an antibody. In certain instances, the antibody is specific for the ECD binding region of the full-length receptor (*e*.*g*., full-length HER2).

In some embodiments, each dilution series of capture antibodies comprises a series of descending capture antibody concentrations. In certain instances, the capture antibodies are serially diluted at least 2-fold (*e.g.,* 2, 5, 10, 20, 50, 100, 500, or 1000-fold) to produce a dilution series comprising a set number (*e.g.,* 2, 3, 4, 5, 6, 7, 8, 9, 10, 15, 20, 25, or more) of descending capture antibody concentrations which are spotted onto an array. Preferably, at least 2, 3, 4, 5, or 6 replicates of each capture antibody dilution are spotted onto the array.

In other embodiments, the solid support comprises glass (*e.g.,* a glass slide), plastic, chips, pins, filters, beads, paper, membrane (*e.g.,* nylon, nitrocellulose, polyvinylidene fluoride (PVDF), *etc*.), fiber bundles, or any other suitable substrate. In a preferred embodiment, the capture antibodies are restrained (*e*.*g*., via covalent or noncovalent interactions) on glass slides coated with a nitrocellulose polymer such as, for example, FAST® Slides, which are commercially available from Whatman Inc. (Florham Park, NJ). Exemplary methods for constructing antibody arrays suitable for use in the invention are described, *e.g.,* in U.S. Patent No. 8,163,499.

### VIII. Production of Antibodies

The generation and selection of antibodies not already commercially available for analyzing the levels of expression and activation of signal transduction molecules in tumor cells in accordance with the immunoassays of the present invention can be accomplished several ways. For example, one way is to express and/or purify a polypeptide of interest (*i*.*e*., antigen) using protein expression and purification methods known in the art, while another way is to synthesize the polypeptide of interest using solid phase peptide synthesis methods known in the art. *See, e.g.,* Guide to Protein Purification, Murray P. Deutcher, ed., Math. Enzymol., Vol. 182 (1990); Solid Phase Peptide Synthesis, Greg B. Fields, ed., Meth. Enzymol., Vol. 289 (1997); Kiso et al., Chem. Pharm. Bull., 38:1192-99 (1990); Mostafavi et al., Biomed Pept. Proteins Nucleic Acids, 1:255-60, (1995); and Fujiwa a et al., Chem. Pharm. Bull., 44:1326-31 (1996). The purified or synthesized polypeptide can then be injected, for example, into mice or rabbits, to generate polyclonal or monoclonal antibodies. One skilled in the art will recognize that many procedures are available for the production of antibodies, for example, as described in Antibodies, A Laboratory Manual, Harlow and Lane, Eds., Cold Spring Harbor Laboratory, Cold Spring Harbor, N.Y. (1988). One skilled in the art will also appreciate that binding fragments or Fab fragments which mimic (*e.g.,* retain the functional binding regions of) antibodies can also be prepared from genetic information by various procedures. *See, e.g*., Antibody Engineering: A Practical Approach, Borrebaeck, Ed., Oxford University Press, Oxford (1995); and Huse et al., J. Immunol., 149:3914-3920 (1992).

Those skilled in the art will recognize that many approaches can be taken in producing antibodies or binding fragments and screening and selecting for affinity and specificity for the various polypeptides of interest, but these approaches do not change the scope of the present invention.

A more detailed description of polyclonal antibodies, monoclonal antibodies, humanized antibodies, human antibodies, bispecific antibodies, fragments thereof, and methods of purifying antibodies is found in U.S. Patent Publication No. 2011/071042.

### IX. Anticancer Therapy

In certain aspects, the anticancer drug comprises an anti-signaling agent (*i*.*e*., a cytostatic drug) such as a monoclonal antibody or a tyrosine kinase inhibitor; an anti-proliferative agent; a chemotherapeutic agent (*i.e.,* a cytotoxic drug); a hormonal therapeutic agent; a radiotherapeutic agent; a vaccine; and/or any other compound with the ability to reduce or abrogate the uncontrolled growth of aberrant cells such as cancerous cells. In some embodiments, the isolated cells are treated with one or more anti-signaling agents, anti-proliferative agents, and/or hormonal therapeutic agents in combination with at least one chemotherapeutic agent. In some embodiments, a patient determined to be at risk for developing CRC is treated with an anticancer drug intended to improve the patient's prognosis for disease-free survival, progression-free survival or overall survival. In other embodiments, a patient determined to be at risk for developing CRC undergoes a polypectomy.

In certain instances, the chemotherapy drugs used in methods of the present invention for colorectal cancer treatment include, but are not limited to, 5-fluorouracil (5-FU), which has been the first-choice chemotherapy drug for colorectal cancer for many years. Other drugs include Camptosar and Eloxatin. Several other chemotherapy drugs also are used for the treatment of colorectal cancer that has spread. These include Vectibix, Erbitux, Avastin and Aflibercept and are usually given along with 5-FU plus Camptosar or Eloxatin for metastatic colorectal cancer. Regorafenib is another drug, that can be taken orally as a single agent after the other drugs have stopped working.

Examples of anti-signaling agents suitable for use in the present invention include, without limitation, monoclonal antibodies such as trastuzumab (Herceptin®), pertuzumab (2C4), alemtuzumab (Campath®), bevacizumab (Avastin®), cetuximab (Erbitux®), gemtuzumab (Mylotarg®), panitumumab (Vectibix™), rituximab (Rituxan®), and tositumomab (BEXXAR®); tyrosine kinase inhibitors such as gefitinib (Iressa®), sunitinib (Sutent®), erlotinib (Tarceva®), lapatinib (GW-572016; Tykerb®), canertinib (CI 1033), semaxinib (SU5416), vatalanib (PTK787/ZK222584), sorafenib (BAY 43-9006; Nexavar®), imatinib mesylate (Gleevec®), leflunomide (SU101), vandetanib (ZACTIMA™; ZD6474), pelitinib, CP-654577, CP-724714, HKI-272, PKI-166, AEE788, BMS-599626, HKI-357, BIBW 2992, ARRY-334543, JNJ-26483327, and JNJ-26483327; and combinations thereof.

Exemplary anti-proliferative agents include mTOR inhibitors such as sirolimus (rapamycin), temsirolimus (CCI-779), everolimus (RAD001), BEZ235, and XL765; AKT inhibitors such as 1L6-hydroxymethyl-chiro-inositol-2-(R)-2-O-methyl-3-O-octadecyl-*sn-*glycerocarbonate, 9-mcthoxy-2-mcthylellipticinium acetate, 1,3-dihydro-1-(1-((4-(6-phenyl-1H-imidazo[4,5-g]quinoxalin-7-yl)phenyl)methyl)-4-piperidinyl)-2H-benzimidazol-2-one, 10-(4'-(N-diethylamino)butyl)-2-chlorophenoxazine, 3-formylchromone thiosemicarbazone (Cu(II)Cl₂ complex), API-2, a 15-mer peptide derived from amino acids 10-24 of the proto-oncogene TCL1 (Hiromura et al., J. Biol. Chem., 279:53407-53418 (2004), KP372-1, and the compounds described in Kozikowski et al., J. Am. Chem. Soc., 125:1144-1145 (2003) and Kau et al., Cancer Cell, 4:463-476 (2003); PI3K inhibitors such as PX-866, wortmannin, LY 294002, quercetin, tetrodotoxin citrate, thioperamide maleate, GDC-0941 (957054-30-7), IC87114, PI-103, PIK93, BEZ235 (NVP-BEZ235), TGX-115, ZSTK474, (-)-deguelin, NU 7026, myricetin, tandutinib, GDC-0941 bismesylate, GSK690693, KU-55933, MK-2206, OSU-03012, perifosine, triciribine, XL-147, PIK75, TGX-221, NU 7441, PI828, XL-765, and WHI-P 154; MEK inhibitors such as PD98059, ARRY-162, RDEA119, U0126, GDC-0973, PD184161, AZD6244, AZD8330, PD0325901, and ARRY-142886; and combinations thereof.

Non-limiting examples of pan-HER inhibitors include PF-00299804, neratinib (HKI-272), AC480 (BMS-599626), BMS-690154, PF-02341066, HM781-36B, CI-1033, BIBW-2992, and combinations thereof.

Non-limiting examples of chemotherapeutic agents include platinum-based drugs (*e.g.,* oxaliplatin, cisplatin, carboplatin, spiroplatin, iproplatin, satraplatin, *etc*.), alkylating agents (*e.g.,* cyclophosphamide, ifosfamide, chlorambucil, busulfan, melphalan, mechlorethamine, uramustine, thiotepa, nitrosoureas, *etc*.), anti-metabolites (*e*.*g*., *5-*fluorouracil, azathioprine, 6-mercaptopurine, methotrexate, leucovorin, capecitabine, cytarabine, floxuridine, fludarabine, gemcitabine (Gemzar®), pemetrexed (ALIMTA®), raltitrexed, *etc.),* plant alkaloids (*e.g.,* vincristine, vinblastine, vinorelbine, vindesine, podophyllotoxin, paclitaxel (Taxol®), docetaxel (Taxotere®), *etc*.), topoisomerase inhibitors (*e.g.,* irinotecan, topotecan, amsacrine, etoposide (VP16), etoposide phosphate, teniposide, *etc.*), antitumor antibiotics (*e.g.,* doxorubicin, adriamycin, daunorubicin, epirubicin, actinomycin, bleomycin, mitomycin, mitoxantrone, plicamycin, *etc*.), pharmaceutically acceptable salts thereof, stereoisomers thereof, derivatives thereof, analogs thereof, and combinations thereof.

Examples of hormonal therapeutic agents include, without limitation, aromatase inhibitors (*e.g.,* aminoglutethimide, anastrozole (Arimidex®), letrozole (Femara®), vorozole, exemestane (Aromasin®), 4-androstene-3,6, 17-trione(6-OXO), 1,4,6-androstatrien-3,17-dione (ATD), formestane (Lentaron®), *etc.*), selective estrogen receptor modulators (*e.g.,* bazedoxifene, clomifene, fulvestrant, lasofoxifene, raloxifene, tamoxifen, toremifene, *etc*.), steroids (*e.g.,* dexamethasone), finasteride, and gonadotropin-releasing hormone agonists (GnRH) such as goserelin, pharmaceutically acceptable salts thereof, stereoisomers thereof, derivatives thereof, analogs thereof, and combinations thereof.

Non-limiting examples of cancer vaccines useful in the present invention include ANYARA from Active Biotech, DCVax-LB from Northwest Biotherapeutics, EP-2101 from IDM Pharma, GV1001 from Pharmexa, IO-2055 from Idera Pharmaceuticals, INGN 225 from Introgen Therapeutics and Stimuvax from Biomira/Merck.

Examples of radiotherapeutic agents include, but are not limited to, radionuclides such as ⁴⁷Sc, ⁶⁴Cu, ⁶⁷Cu, ⁸⁹Sr, ⁸⁶Y, ⁸⁷Y, ⁹⁰Y, ¹⁰⁵Rh, ¹¹¹Ag, ¹¹¹In, ^{117m}Sn, ¹⁴⁹Pm, ¹⁵³Sm, ¹⁶⁶Ho, ¹⁷⁷Lu, ¹⁸⁶Re, ¹⁸⁸Re, ²¹¹At, and ²¹²Bi, optionally conjugated to antibodies directed against tumor antigens.

Non-limiting examples of HER2 inhibitors include monoclonal antibodies such as trastuzumab (Herceptin®) and pertuzumab (2C4); small molecule tyrosine kinase inhibitors such as gefitinib (Iressa®), erlotinib (Tarceva®), pelitinib, CP-654577, CP-724714, canertinib (CI 1033), HKI-272, lapatinib (GW-572016; Tykerb®), PKI-166, AEE788, BMS-599626, HKI-357, BIBW 2992, ARRY-334543, JNJ-26483327, and JNJ-26483327; and combinations thereof.

Non-limiting examples of c-Met inhibitors include monoclonal antibodies such as AMG102 and MetMAb; small molecule inhibitors of c-Met such as ARQ197, JNJ-38877605, PF-04217903, SGX523, GSK 1363089/XL880, XL184, MGCD265, and J\1K-2461; and combinations thereof.

### X. Methods of Administration

According to the to the present disclosure the anticancer drugs described herein are administered to a subject by any convenient means known in the art. The methods of the present disclosure can be used to select a suitable anticancer drug or combination of anticancer drugs for the treatment of a tumor, *e.g.,* a colorectal tumor, in a subject. One skilled in the art will appreciate that the anticancer drugs described herein can be administered alone or as part of a combined therapeutic approach with conventional chemotherapy, radiotherapy, hormonal therapy, immunotherapy, and/or surgery.

In certain aspects, the anticancer drug comprises an anti-signaling agent (*i*.*e*., a cytostatic drug) such as a monoclonal antibody or a tyrosine kinase inhibitor; an anti-proliferative agent; a chemotherapeutic agent (*i.e*., a cytotoxic drug); a hormonal therapeutic agent; a radiotherapeutic agent; a vaccine; and/or any other compound with the ability to reduce or abrogate the uncontrolled growth of aberrant cells such as cancerous cells. In some aspects, the subject is treated with one or more anti-signaling agents, anti-proliferative agents, and/or hormonal therapeutic agents in combination with at least one chemotherapeutic agent. Exemplary monoclonal antibodies, tyrosine kinase inhibitors, anti-proliferative agents, chemotherapeutic agents, hormonal therapeutic agents, radiotherapeutic agents, and vaccines are described above.

In some aspects, the anticancer drugs described herein can be co-administered with conventional immunotherapeutic agents including, but not limited to, immunostimulants (*e*.*g*., Bacillus Calmette-Guerin (BCG), levamisole, interleukin-2, alpha-interferon, *etc*.), immunotoxins (*e.g.,* anti-CD33 monoclonal antibody-calicheamicin conjugate, anti-CD22 monoclonal antibody-pseudomonas exotoxin conjugate, *etc*.), and radioimmunotherapy (*e*.*g.,* anti-CD20 monoclonal antibody conjugated to ¹¹¹In, ⁹⁰Y, or ¹³¹I, *etc.*).

Anticancer drugs can be administered with a suitable pharmaceutical excipient as necessary and can be carried out via any of the accepted modes of administration. Thus, administration can be, for example, oral, buccal, sublingual, gingival, palatal, intravenous, topical, subcutaneous, transcutaneous, transdermal, intramuscular, intra-joint, parenteral, intra-arteriole, intradermal, intraventricular, intracranial, intraperitoneal, intravesical, intrathecal, intralesional, intranasal, rectal, vaginal, or by inhalation. By "co-administer" it is meant that an anticancer drug is administered at the same time, just prior to, or just after the administration of a second drug (*e.g.,* another anticancer drug, a drug useful for reducing the side-effects associated with anticancer drug therapy, a radiotherapeutic agent, a hormonal therapeutic agent, an immunotherapeutic agent, *etc*.).

A therapeutically effective amount of an anticancer drug may be administered repeatedly, *e.g.,* at least 2, 3, 4, 5, 6, 7, 8, or more times, or the dose may be administered by continuous infusion. The dose may take the form of solid, semi-solid, lyophilized powder, or liquid dosage forms, such as, for example, tablets, pills, pellets, capsules, powders, solutions, suspensions, emulsions, suppositories, retention enemas, creams, ointments, lotions, gels, aerosols, foams, or the like, preferably in unit dosage forms suitable for simple administration of precise dosages.

As used herein, the term "unit dosage form" refers to physically discrete units suitable as unitary dosages for human subjects and other mammals, each unit containing a predetermined quantity of an anticancer drug calculated to produce the desired onset, tolerability, and/or therapeutic effects, in association with a suitable pharmaceutical excipient (*e.g*., an ampoule). In addition, more concentrated dosage forms may be prepared, from which the more dilute unit dosage forms may then be produced. The more concentrated dosage forms thus will contain substantially more than, *e.g.,* at least 1, 2, 3, 4, 5, 6, 7, 8, 9, 10, or more times the amount of the anticancer drug.

Methods for preparing such dosage forms are known to those skilled in the art (*see, e.g.,* REMINGTON'SPHARAMCEUTICAL SCIENCES, 18TH ED., Mack Publishing Co., Easton, PA (1990)). The dosage forms typically include a conventional pharmaceutical carrier or excipient and may additionally include other medicinal agents, carriers, adjuvants, diluents, tissue permeation enhancers, solubilizers, and the like. Appropriate excipients can be tailored to the particular dosage form and route of administration by methods well known in the art (*see, e.g*., *REMINGTON'S PHARMACEUTICAL SCIENCES, supra*).

Examples of suitable excipients include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline, syrup, methylcellulose, ethylcellulose, hydroxypropylmethylcellulose, and polyacrylic acids such as Carbopols, *e.g.,* Carbopol 941, Carbopol 980, Carbopol 981, *etc.* The dosage forms can additionally include lubricating agents such as talc, magnesium stearate, and mineral oil; wetting agents; emulsifying agents; suspending agents; preserving agents such as methyl-, ethyl-, and propyl-hydroxy-benzoates (*i.e*., the parabens); pH adjusting agents such as inorganic and organic acids and bases; sweetening agents; and flavoring agents. The dosage forms may also comprise biodegradable polymer beads, dextran, and cyclodextrin inclusion complexes.

For oral administration, the therapeutically effective dose can be in the form of tablets, capsules, emulsions, suspensions, solutions, syrups, sprays, lozenges, powders, and sustained-release formulations. Suitable excipients for oral administration include pharmaceutical grades of mannitol, lactose, starch, magnesium stearate, sodium saccharine, talcum, cellulose, glucose, gelatin, sucrose, magnesium carbonate, and the like.

In some aspects, the therapeutically effective dose takes the form of a pill, tablet, or capsule, and thus, the dosage form can contain, along with an anticancer drug, any of the following: a diluent such as lactose, sucrose, dicalcium phosphate, and the like; a disintegrant such as starch or derivatives thereof; a lubricant such as magnesium stearate and the like; and a binder such a starch, gum acacia, polyvinylpyrrolidone, gelatin, cellulose and derivatives thereof. An anticancer drug can also be formulated into a suppository disposed, for example, in a polyethylene glycol (PEG) carrier.

Liquid dosage forms can be prepared by dissolving or dispersing an anticancer drug and optionally one or more pharmaceutically acceptable adjuvants in a carrier such as, for example, aqueous saline (*e*.*g*., 0 9% w/v sodium chloride), aqueous dextrose, glycerol, ethanol, and the like, to form a solution or suspension, *e.g.,* for oral, topical, or intravenous administration. An anticancer drug can also be formulated into a retention enema.

For topical administration, the therapeutically effective dose can be in the form of emulsions, lotions, gels, foams, creams, jellies, solutions, suspensions, ointments, and transdermal patches. For administration by inhalation, an anticancer drug can be delivered as a dry powder or in liquid form via a nebulizer. For parenteral administration, the therapeutically effective dose can be in the form of sterile injectable solutions and sterile packaged powders. Preferably, injectable solutions are formulated at a pH of from about 4.5 to about 7.5.

The therapeutically effective dose can also be provided in a lyophilized form. Such dosage forms may include a buffer, *e.g.,* bicarbonate, for reconstitution prior to administration, or the buffer may be included in the lyophilized dosage form for reconstitution with, *e.g.,* water. The lyophilized dosage form may further comprise a suitable vasoconstrictor, *e.g.,* epinephrine. The lyophilized dosage form can be provided in a syringe, optionally packaged in combination with the buffer for reconstitution, such that the reconstituted dosage form can be immediately administered to a subject.

A subject can also be monitored at periodic time intervals to assess the efficacy of a certain therapeutic regimen. For example, the activation states of certain signal transduction molecules may change based on the therapeutic effect of treatment with one or more of the anticancer drugs described herein. The subject can be monitored to assess response and understand the effects of certain drugs or treatments in an individualized approach. Additionally, subjects who initially respond to a specific anticancer drug or combination of anticancer drugs may become refractory to the drug or drug combination, indicating that these subjects have developed acquired drug resistance. These subjects can be discontinued on their current therapy and an alternative treatment prescribed.

### XI. Example

The following example is offered to illustrate, but not to limit, the claimed invention.

### Example 1. High Levels of Activated Analytes Used in a Patient's Polyp Sample for Diagnosing Colorectal Cancer

This example shows a method of determining the risk of developing colorectal cancer in an individual. The method includes detecting the activation and/or expression level of at least one signal transduction molecule in a cell lysate prepared from a polyp sample taken from the individual, comparing the activation and/or expression level to a control, and indicating that the individual is at risk of developing colorectal cancer if the activation and/or expression level of the sample is higher than that of a reference control (*e*.*g*., a healthy control or a non- adenomatous polyp control). This example provides an exemplary embodiment of the method using patient samples from a study of polyp recurrence and progression in colorectal cancer.

Patients included in the study had undergone a polypectomy in and/or after 2003, were affected by multiple polyps < 10 mm or one to two adenomas < 10 mm and/or with a grade of dysplasia to make them classified at intermediate risk for CRC, and are scheduled for a screening colonoscopy every 3-5 years.

Eight biopsy samples per patient were collected from the non-adenomatous mucosa in the sigmoidal colon. Small polyps such as those with a diameter less than or equal to 0.5 cm were topically electrocoagulated. Villous and tubulovillous polyps such as those with a diameter equal to or higher than 0.5 cm were submitted for histological assessment. Patients' bowels were cleansed by oral administration of a PEG-4000 solution (*e.g.,* 1, 120g PEG 4000/ 4 L of water) prior to sample biopsy. In total 42 human tissue biopsy samples of about 2-3 mm in size were collected.

Tissue lysates were prepared using the MSD® Tris Lysis Buffer (Meso Scale Discovery, Gaithersburg, MD) or a lysis buffer (Prometheus Laboratories, San Diego, CA).

The activation (phosphorylation) level and/or expression levels of analytes including HER1, HER2, HER3, cMET, PI3K, IGF1R, SHC, CK, Akt, Erk, Mek, Rsk, PRAS and RPS6 were measured using CEER assays.

For each assay, two lysate concentrations were used (*e.g.,* 8 µg and 2 µg as shown in FIG. IB). Typically, 1 µg of cell lysate contains about 200 to 400 cells. Specific amounts of sample were used for each particular analyte assay.

The data presented herein represents the analytes that gave a significant activated signal above the lower limit of quantification (LLOQ). The analytes that did not give a significant result were omitted from the data analysis.

The patient data was separated into two groups according to clinical data from tissue biopsies: patients that were normal vs. patients with polyps. Table 1 lists representative patient samples used in the study and the clinical descriptions of the patients. The data of group with polyps was further sorted into two subsets according to the level of phosphorylated HER3. The "Polyps (Group A)" group included patients with a level of HER3 activation similar to normal. The "Polyps (Group B)" group included patients with a high level of HER3 activation.

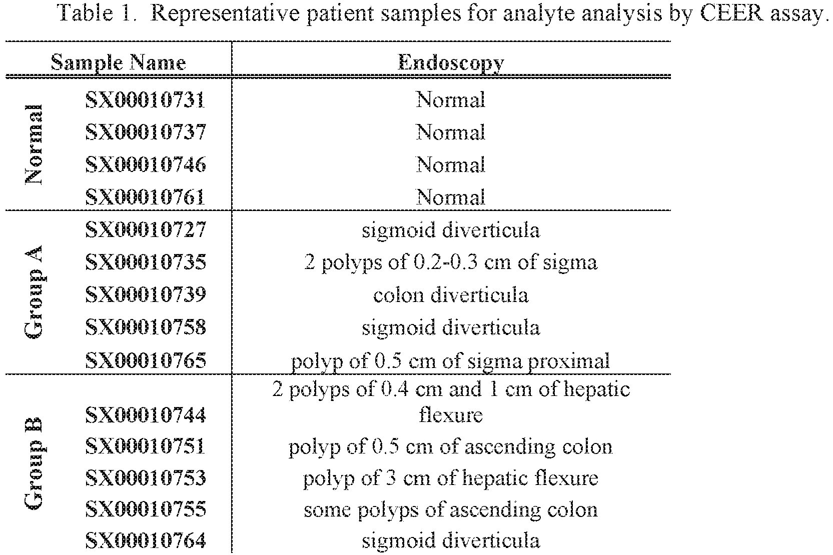

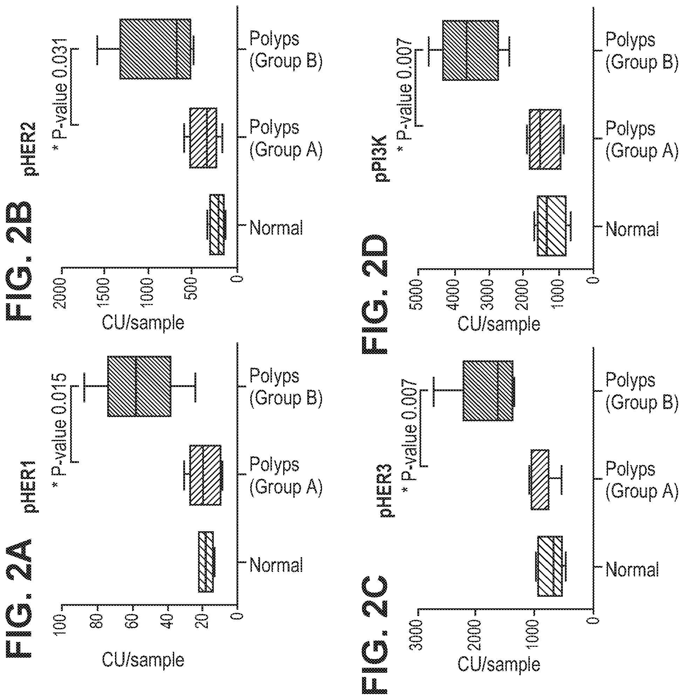

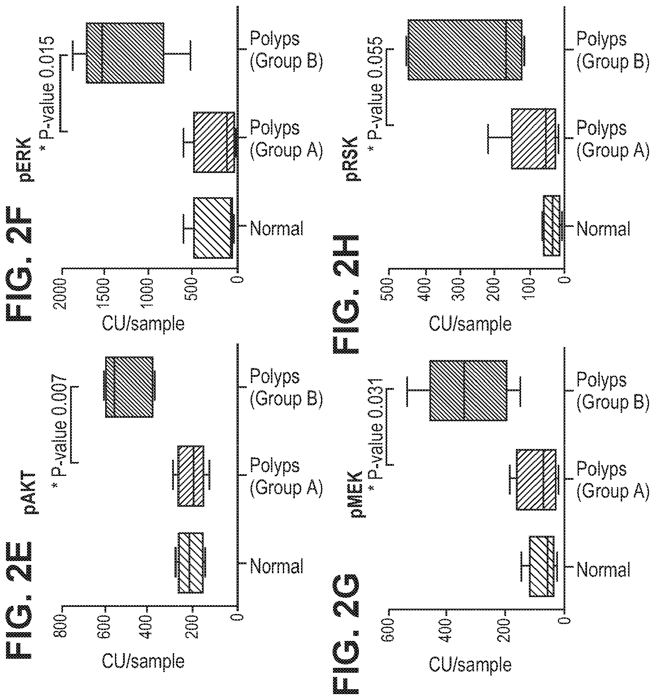

The analyte data shows that activation levels of HER1, HER2, HER3, PI3K, AKT, ERK, MEK and RSK of Group B were statistically significantly higher than those of Group A. Thus, it has been contemplated that the presence of high levels of activated analytes in a polyp sample taken from a subject can indicate that the subject is at risk of developing cancer.
The graphs of FIG. 2 also show that the activated levels of HER1, HER2, HER3, PI3K, AKT, ERK, MEK and RSK are similar between the normal patients and patients of Group A. Total PI3K levels were used as a sample loading control for all of the analytes measured. Table 2 shows the data from normal patient samples used in the analysis. The data numbers represent computed units (CUs) per sample.

**Table 2. Levels of activated analytes in samples from normal patients (e.g., healthy, control patients).**

| **Sample Name** | pAKT | pERK | pMEK | pRSK | pHER 1 | pHER 2 | pHER 3 | pPI3K | tPI3K |
|---|---|---|---|---|---|---|---|---|---|
| **SX0001073 1** | 194 | 175 | 58 | 13 | 20 | 176 | 484 | 1661 | 987 |
| **SX0001073 7** | 142 | 66 | 49 | 63 | 74 | 321 | 591 | 649 | 2529 |
| **SX0001074 6** | 272 | 944 | 142 | 27 | 16 | 195 | 755 | 1495 | 2286 |
| **SX0001076 1** | 23 6 | 55 | 26 | 44 | 13 | 134 | 970 | 1299 | 1866 |
| **Average** | 211 | 310 | 69 | 37 | 31 | 207 | 700 | 1276 | 1917 |

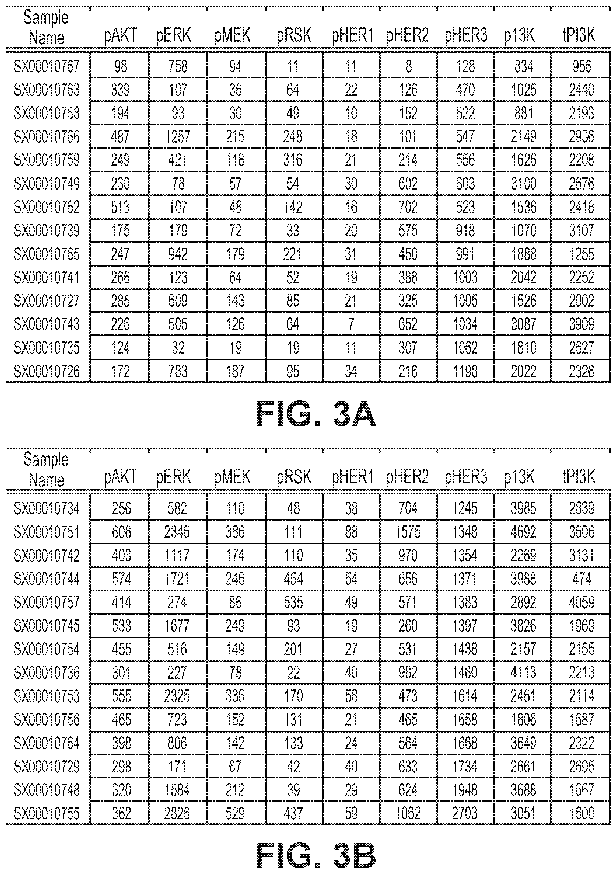

FIG. 3 shows that levels of the activated analytes in samples from Group A and Group B. The data numbers represent computed units (CU) per sample. The groups show a distinctive pattern of activated cancer pathway markers (*e.g.,* pAKT, pERK, pMEK, pRSK, pHER1, pHER2, pHER3, and pPI3K). In particular, Group A in FIG. 3A exhibited a lower level of activation for the assayed analytes. Group B in FIG. 3B exhibited a higher level of activation for the assayed analytes. Total PI3K was used as a loading control to show an equal amount of protein is present in the samples.

The receptor tyrosine kinases HER 1, HER 2 and HER 3 are well documented in the literature and the clinical setting as being involved in various cancer malignancies. It has been demonstrated that high HER 3 phosphorylation correlates with phosphorylated PI3K. Data of the present study (*see,* FIG. 3) also show a direct correlation between these two markers. In addition, when PI3K levels are high, AKT, a direct downstream target of PI3K, also increases (*see,* FIG. 3). As expected since MEK is driven by HER1 and HER2, high activated HER 1 and HER 2 levels trended with high phospho MEK levels. Moreover, phospho ERK and RSK which are downstream of MEK were also highly activated in the Group B polyps. Taken together, the data demonstrate that patients in Group B have polyps express activated markers that are associated with cancer pathways.

The data presented in this example show that the levels of activated markers in cancer pathways such as those of the HER1, HER2, HER3, PI3K, and MEK pathways are associated with a particular subset of polyps. The presence of such polyps in a subject indicate that the subjects is at risk of developing colorectal cancer. Thus, it has been contemplated that the presence of higher levels of activated analytes in a patient's polyp sample taken can be used to diagnose the patient as having CRC or as a propensity of developing CRC. The method described herein provides an assay for early detection of the risk of developing CRC in pre-cancerous polyp or benign polyp samples.

### Methods

### Multiplexed microarray printing for CEER assay

Capture Abs were printed on nitrocellulose-coated glass slides (ONCYTE, Grace Biolab) using non-contact printers (NanoPlotter, GeSim). The spot diameter was approximately 175 µm and printed slides were kept in a desiccated chamber at room temperature. Approximately 500 pl (picoliters) of capture Abs were printed in triplicate and at serial dilution concentrations of 1 mg/ml and 0.5 mg/ml. Purified mouse-IgGs were printed as a negative control. Analytical calibration reactions and internal quality control reactions were run on every slide.

### Antibody conjugation and purification for CEER assay

Target specific-Abs and corresponding detector enzymes were activated with a bifunctional cross-linker, succinimidyl-4-(N-maleimidomethyl)cyclohexane-1-carboxylate (SMCC). The conjugate was purified by HPLC using a size-exclusion column. The antibody activities in the purified conjugates were detected by competition ELISA and enzyme activity was detected by a functional assay specific for each detector enzyme.

### Performing CEER assay

Slides spotted with antibodies were blocked in 80 µl of Blocking Buffer for 1 hour at room temperature, followed by 2 washes with TBST. Afterwards, 80 µl of serial diluted sample lysates, standards, and controls were added to each corresponding slides, followed by overnight incubation at room temperature. The next day, slides were washed 5 times with TBST and antibodies specific for either the phospho analyte or the total analyte (*e.g.,* phosphor-HER2 or total HER2) were added to the slides, followed by a 2 hr incubation at room temperature. Tyramide solution was then added to mediate signal generation and amplification in the presence of glucose. Subsequent incubation with Alexa Fluors dye resulted in the development of a fluorescence signal. After drying, the slides were scanned on a scanner (TECAN, San Jose, CA) and the captured data was further analyzed using the ProScan Software and plotted using a quantitation algorithm (Prometheus Laboratories, San Diego, CA).

### Assay Standards for CEER

A mixed cell lysate containing BT474, T47D and HCC827 was used for HER1, HER2, HER3, cMET and P13K quantitation. Specifically, BT474 cells were used as the standard for HER2; T47D cells were used as the standard for HER3; and HCC827 cells were used as the standard for HER1, cMET and PI3K. Each standard consists of an optimal cell titration as previously determined. Each analyte reference standard was analyzed along with its corresponding reference sample (*e*.*g*., HER2 analyte with BT474 cells) to generate a computed unit (CU) such that 1 CU is equal to the signal generated from the lysate of one reference cell. Each analysis included a buffer control for background subtraction. Recombinant proteins were used as control for the following analytes: AKT, PRAS40, RPS6, MEK, and ERK. Captured data were quantitated using algorithm developed by Prometheus.

This example provide evidence of the use of a highly sensitive assay for colorectal cancer that requires a minimal amount of tissue sample to analyze the expression of biomarkers known to be up-regulated in many types of cancer.

## Claims

1. A method for diagnosing the risk of developing colorectal cancer (CRC) in a subject or identifying a subject as likely to develop colorectal cancer (CRC), the method comprising:
a) lysing a cell from a polyp sample taken from the subject to form a cell lysate;
b) measuring the activation and/or expression level of the at least one signal transduction analyte in the cell lysate, wherein the at least one signal transduction analyte is HER1; and
c) indicating whether the subject is at risk of developing CRC based upon the activation and/or expression level of the at least one signal transduction analyte compared to that of a control.

2. The method of claim **1,** wherein the at least one signal transduction analyte further includes one of more signal transduction analytes selected from the group consisting of HER2, HER3, cMET, PI3K, IGF1R, SHC, CK, AKT, ERK, MEK, RSK, PRAS, RPS6, and a combination thereof.

3. The method of any one of claims **1-2,** wherein the subject is at risk of developing CRC when the measured activation and/or expression level of the at least one signal transduction analyte is higher compared to that of a control.

4. The method of any one of claims **1-3,** wherein step (b) comprises measuring the activation and/or expression level of any combination of two, three, four, five, six, seven, eight, nine, ten, eleven, twelve, thirteen, or fourteen of said signal transduction analyte.

5. The method of any one of claims **1-4,** wherein step (b) is performed with a proximity dual detection assay.

6. The method of any one of claims **1-5,** wherein the proximity dual detection assay is a Collaborative Enzyme Enhanced Reactive ImmunoAssay (CEER).

7. The method of any one of claims **1-6,** wherein the activation level of the at least one signal transduction analyte corresponds to the phosphorylation level thereof.

8. The method of any one of claims **1-7,** wherein the activation level of the at least one signal transduction analyte corresponds to the phosphorylation level of HER1, HER2, HER3, cMET, PI3K, IGF1R, SHC, CK, AKT, ERK, MEK, RSK, PRAS, or RPS6.

9. The method of any one of claims **1-8,** wherein the activation level of the at least one signal transduction analyte corresponds to a level of a PI3K complex.

10. The method of any one of claims **1-9,** wherein the control is from a non-adenomatous tissue.

11. The method of any one of claims **1-10,** wherein the polyp sample is an adenomatous polyp.

12. The method of any one of claims **1-11,** wherein the control is from a healthy subject or CRC subject.

13. The method of any one of claims **1-12,** wherein the polyp sample is from a polypectomy.

14. The method of claim **13,** wherein the polypectomy is a polypectomy of the colon and/or rectum.

15. The method for identifying a subject as likely to develop colorectal cancer (CRC) of claim 1, the method comprising:
measuring the activation and/or expression level of at least one signal transduction analyte in a cell lysate obtained from a polyp sample taken from the subject, wherein the subject is likely to develop CRC when the measured activation and/or expression level of the at least one signal transduction analyte is higher compared to that of a control.

## Patentansprüche

1. Verfahren zum Diagnostizieren des Risikos der Entwicklung von Kolorektalkrebs (CRC) in einem Individuum oder Identifizieren eines Individuums, das wahrscheinlich Kolorektalkrebs (CRC) entwickelt, wobei das Verfahren umfasst:
a) Lysieren einer Zelle aus einer Polyp-Probe, die dem Individuum entnommen wurde, um ein Zelllysat zu bilden;
b) Messen des Aktivierungs- und/oder Expressionsniveaus des mindestens einen Signaltransduktionsanalyten in dem Zelllysat, wobei der mindestens eine Signaltransduktionsanalyt HER1 ist; und
c) Anzeigen, ob für das Individuum das Risiko besteht, CRC zu entwickeln, basierend auf dem Aktivierungs- und/oder Expressionsniveau des mindestens einen Signaltransduktionsanalyten im Vergleich zu dem einer Kontrolle.

2. Verfahren nach Anspruch **1,** wobei der mindestens eine Signaltransduktionsanalyt ferner einen oder mehrere Signaltransduktionsanalyten umfasst, ausgewählt aus der Gruppe, bestehend aus HER2, HER3, cMET, PI3K, IGF1R, SHC, CK, AKT, ERK, MEK, RSK, PRAS, RPS6 und einer Kombination davon.

3. Verfahren nach einem der Ansprüche **1** bis **2**, wobei für das Individuum das Risiko besteht, CRC zu entwickeln, wenn das gemessene Aktivierungs- und/oder Expressionsniveau des mindestens einen Signaltransduktionsanalyten im Vergleich zu dem einer Kontrolle höher ist.

4. Verfahren nach einem der Ansprüche **1** bis **3,** wobei Schritt (b) das Messen des Aktivierungs- und/oder Expressionsniveaus einer beliebigen Kombination von zwei, drei, vier, fünf, sechs, sieben, acht, neun, zehn, elf, zwölf, dreizehn oder vierzehn des Signaltransduktionsanalyten umfasst.

5. Verfahren nach einem der Ansprüche **1** bis **4**, wobei Schritt (b) mit einem Proximity Dual Detection Assay durchgeführt wird.

6. Verfahren nach einem der Ansprüche 1 bis **5,** wobei der Proximity Dual Detection Assay ein Collaborative Enzyme Enhanced Reactive Immunoassay (CEER) ist.

7. Verfahren nach einem der Ansprüche **1** bis **6**, wobei das Aktivierungsniveau des mindestens einen Signaltransduktionsanalyten dem Phosphorylierungsniveau desselben entspricht.

8. Verfahren nach einem der Ansprüche **1** bis **7**, wobei das Aktivierungsniveau des mindestens einen Signaltransduktionsanalyten dem Phosphorylierungsniveau von HER1, HER2, HER3, cMET, PI3K, IGF1R, SHC, CK, AKT, ERK, MEK, RSK, PRAS oder RPS6 entspricht.

9. Verfahren nach einem der Ansprüche **1** bis **8,** wobei das Aktivierungsniveau des mindestens einen Signaltransduktionsanalyten einem Niveau eines P13K-Komplexes entspricht.

10. Verfahren nach einem der Ansprüche **1** bis **9**, wobei die Kontrolle aus einem nicht adenomatösen Gewebe stammt.

11. Verfahren nach einem der Ansprüche **1** bis **10**, wobei die Polyp-Probe ein adenomatöser Polyp ist.

12. Verfahren nach einem der Ansprüche **1** bis **11,** wobei die Kontrolle von einem gesunden Individuum oder einem CRC-Individuum stammt.

13. Verfahren nach einem der Ansprüche **1** bis **12**, wobei die Polyp-Probe aus einer Polypektomie stammt.

14. Verfahren nach Anspruch **13,** wobei die Polypektomie eine Polypektomie des Kolons und/oder Rektums ist.

15. Verfahren zum Identifizieren eines Individuums, das wahrscheinlich Kolorektalkrebs (CRC) entwickelt, nach Anspruch 1, wobei das Verfahren umfasst:
Messen des Aktivierungs- und/oder Expressionsniveaus mindestens eines Signaltransduktionsanalyten in einem Zelllysat, das aus einer Polyp-Probe erlangt wird, die dem Individuum entnommen wurde, wobei das Individuum wahrscheinlich CRC entwickelt, wenn das gemessene Aktivierungs- und/oder Expressionsniveau des mindestens einen Signaltransduktionsanalyten höher ist als das einer Kontrolle.

## Revendications

1. Procédé destiné au diagnostic du risque de développer un cancer colorectal (CRC) chez un sujet ou à l'identification d'un sujet comme susceptible de développer un cancer colorectal (CRC), le procédé comprenant :
a) la lyse d'une cellule à partir d'un échantillon de polype prélevé chez le sujet pour former un lysat cellulaire ;
b) la mesure du niveau d'activation et/ou d'expression d'au moins un analyte de transduction de signal dans le lysat cellulaire, dans lequel ledit au moins un analyte de transduction de signal est HER1 ; et
c) le fait d'indiquer si le sujet présente un risque de développer un CRC sur la base du niveau d'activation et/ou d'expression dudit au moins un analyte de transduction de signal par rapport à celui d'un témoin.

2. Procédé selon la revendication **1,** dans lequel ledit au moins un analyte de transduction de signal comprend en outre un ou plusieurs analytes de transduction de signal choisis dans le groupe constitué par HER2, HER3, cMET, PI3K, IGF1R, SHC, CK, AKT, ERK, MEK, RSK, PRAS, RPS6 et une combinaison de ceux-ci.

3. Procédé selon l'une quelconque des revendications **1 à 2,** dans lequel le sujet présente un risque de développer un CRC lorsque le niveau d'activation et/ou d'expression mesuré dudit au moins un analyte de transduction de signal est supérieur par rapport à celui d'un témoin.

4. Procédé selon l'une quelconque des revendications **1 à 3,** dans lequel l'étape (b) comprend la mesure du niveau d'activation et/ou d'expression d'une combinaison quelconque de deux, trois, quatre, cinq, six, sept, huit, neuf, dix, onze, douze, treize ou quatorze dudit analyte de transduction de signal.

5. Procédé selon l'une quelconque des revendications **1 à 4,** dans lequel l'étape (b) est effectuée avec un essai de détection double de proximité.

6. Procédé selon l'une quelconque des revendications **1 à 5,** dans lequel l'essai de détection double de proximité est un essai immunologique réactif collaboratif amélioré par une enzyme (CEER).

7. Procédé selon l'une quelconque des revendications **1 à 6,** dans lequel le niveau d'activation dudit au moins un analyte de transduction de signal correspond au niveau de phosphorylation de celui-ci.

8. Procédé selon l'une quelconque des revendications **1 à 7,** dans lequel le niveau d'activation dudit au moins un analyte de transduction de signal correspond au niveau de phosphorylation de HER1, HER2, HER3, cMET, PI3K, IGF1R, SHC, CK, AKT, ERK, MEK, RSK, PRAS ou RPS6.

9. Procédé selon l'une quelconque des revendications **1 à 8,** dans lequel le niveau d'activation dudit au moins un analyte de transduction de signal correspond à un niveau d'un complexe de PI3K.

10. Procédé selon l'une quelconque des revendications **1 à 9,** dans lequel le témoin provient d'un tissu non adénomateux.

11. Procédé selon l'une quelconque des revendications **1 à 10,** dans lequel l'échantillon de polype est un polype adénomateux.

12. Procédé selon l'une quelconque des revendications **1 à 11,** dans lequel le témoin provient d'un sujet sain ou d'un sujet souffrant d'un CRC.

13. Procédé selon l'une quelconque des revendications **1 à 12,** dans lequel l'échantillon de polype provient d'une polypectomie.

14. Procédé selon la revendication **1**3, dans lequel la polypectomie est une polypectomie du côlon et/ou du rectum.

15. Procédé destiné à l'identification d'un sujet comme étant susceptible de développer un cancer colorectal (CRC) selon la revendication 1, le procédé comprenant :
la mesure du niveau d'activation et/ou d'expression d'au moins un analyte de transduction du signal dans un lysat cellulaire obtenu à partir d'un échantillon de polype prélevé chez le sujet, le sujet étant susceptible de développer un CRC lorsque le niveau d'activation et/ou d'expression mesuré dudit au moins un analyte de transduction de signal est supérieur par rapport à celui d'un témoin.
